# EUROPEAN PATENT APPLICATION

(11) **EP 4 501 927 A1**
(43) Date of publication of application: **05.02.2025**
(21) Application number: 23774003.0
(22) Date of filing: 24.03.2023
(51) Int. Cl.: C07D 409/14, C07D 249/18, A61K 31/4709, A61P 11/00

(54) **CRYSTAL OF FUSED TRICYCLIC DERIVATIVE OR PHARMACEUTICALLY ACCEPTABLE SALT THEREOF**

(30) Priority: 25.03.2022 CN 202210307797
(71) Applicant: Chia Tai Tianqing Pharmaceutical Group Co., Ltd., Lianyungang, Jiangsu 222062 (CN)
(72) Inventor: GAO, Feng, Shanghai 200131 (CN); YAO, Ting, Shanghai 200131 (CN); ZHANG, Peng, Shanghai 200131 (CN); LUO, Yunfu, Shanghai 200131 (CN)
(74) Representative: Potter Clarkson
(86) International application number: PCT/CN2023/083637
(87) International publication number: WO 2023/179758

(57) **Abstract**

Disclosed in the present application is a crystal of a fused tricyclic derivative or a pharmaceutically acceptable salt thereof; and specifically disclosed are a crystal of a compound of formula (I), a crystal of a pharmaceutically acceptable salt thereof, and a preparation method therefor and the use thereof.

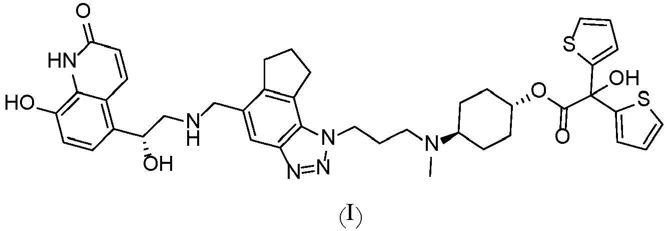

## Description

### The present application claims the following priority:

The present application claims the benefit and priority to the Chinese Patent Application No.2022103077974 filed with National Intellectual Property Administration, PRC on March 25, 2022, which is incorporated herein by reference in its entirety.

### TECHNICAL FIELD

The present application relates to a crystal of a fused tricyclic derivative or a pharmaceutically acceptable salt thereof, a preparation method therefor, and use of the crystal in the manufacture of a medicament for treating a disease related to M3 receptor and β2 receptor.

### BACKGROUND

Bronchodilators play an important role in the treatment of respiratory diseases (e.g., chronic obstructive pulmonary disease and asthma).β-adrenergic receptor agonists and muscarinic receptor antagonists are well-established bronchodilators in a wide range of clinical applications. At present, β-adrenergic receptor agonists used by the inhalation route include short-acting drugs such as salbutamol and terbutaline, and long-acting drugs such as salmeterol, formoterol, vilanterol, and indacaterol.These drugs produce bronchodilation by stimulating adrenergic receptors on airway smooth muscle and reverse the response of bronchoconstrictors to various mediators (e.g., acetylcholine). Currently used inhaled muscarinic antagonists include short-acting drugs such as ipratropium or oxitropium, and long-acting drugs such as tiotropium. These drugs can produce bronchodilation by reducing vagal cholinergic activity in airway smooth muscle. In addition to improving lung function, these drugs can also improve quality of life and reduce exacerbations. Clinical practice has shown that the combination of β2 agonists and M3 antagonists is more effective for the treatment of chronic obstructive pulmonary disease (COPD) than either component alone.At present, muscarinic receptor antagonists and β2 adrenergic receptor agonists are prepared into compound formulations for the treatment of asthma and moderate to severe COPD. Such compound formulation mainly includes Anoro Ellipta (umeclidinium/vilanterol), Ultibro Breezhaler (glycopyrronium/indacaterol) and Duaklir (aclidinium bromide + formoterol fumarate), etc. Although compound formulations have better therapeutic effects than any single preparation, they have higher requirements in formulation preparation.

A single molecule with dual activity at M3 muscarinic receptor and β2 adrenoceptor (MABA) will be more inclined to exhibit a synergistic drug effect on a target in the case of consistent pharmacokinetic properties, and will also offer relevant advantages in formulation as compared to a two-component combination. It will also be easier to formulate with other therapeutic agents (such as inhaled corticosteroids) to provide a triple therapy combination. Therefore, a novel drug having both β2 receptor agonist and muscarinic receptor antagonist activities and suitable for the treatment of respiratory diseases such as asthma and COPD is of high clinical value and interest.

### SUMMARY

The present application provides a crystal form of a compound of formula (I),

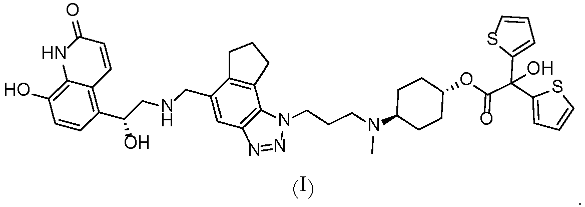

The present application provides a crystal form A of the compound of formula (I), 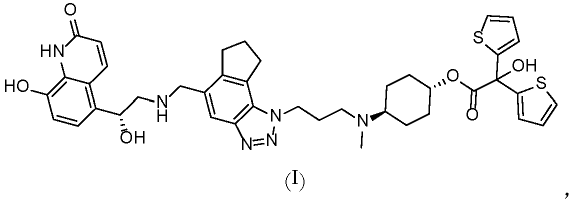 wherein the crystal form A has an X-ray powder diffraction pattern comprising diffraction peaks, in terms of 2Θ angle, at 12.65±0.20° and 24.06±0.20°.

In some embodiments of the present application, in an X-ray powder diffraction (XRPD) pattern of the crystal form A of the compound of formula (I), the peak positions and relative intensities of diffraction peaks are shown in Table 1:

**Table 1. Peak positions and relative intensities of diffraction peaks in the XRPD pattern of the crystal form A**

| No. | 2θ angle (±0.20°) | Relative intensity (%) |
|---|---|---|
| 1 | 12.65 | 10000 |
| 2 | 24.06 | 42.56 |

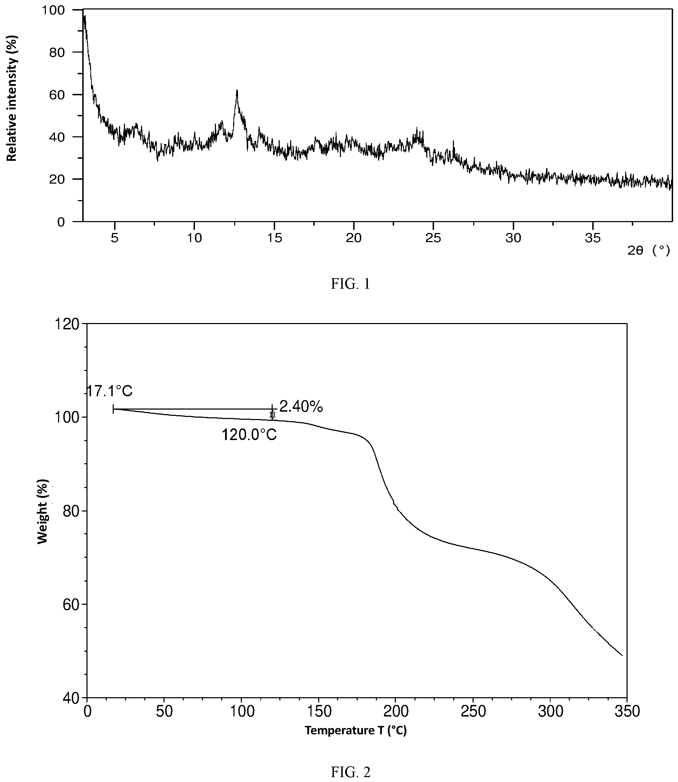

In some embodiments of the present application, the crystal form A of the compound of formula (I) has an X-ray powder diffraction pattern as shown in FIG. 1.

In some embodiments of the present application, the crystal form A of the compound of formula (I) has a thermogravimetric analysis (TGA) curve showing a weight loss of 2.40% at 120.0°C.

In some embodiments of the present application, the crystal form A of the compound of formula (I) has a TGA pattern as shown in FIG. 2.

In some embodiments of the present application, the crystal form A of the compound of formula (I) has a differential scanning calorimetry (DSC) curve showing an endothermic peak starting point at 140.5°C.

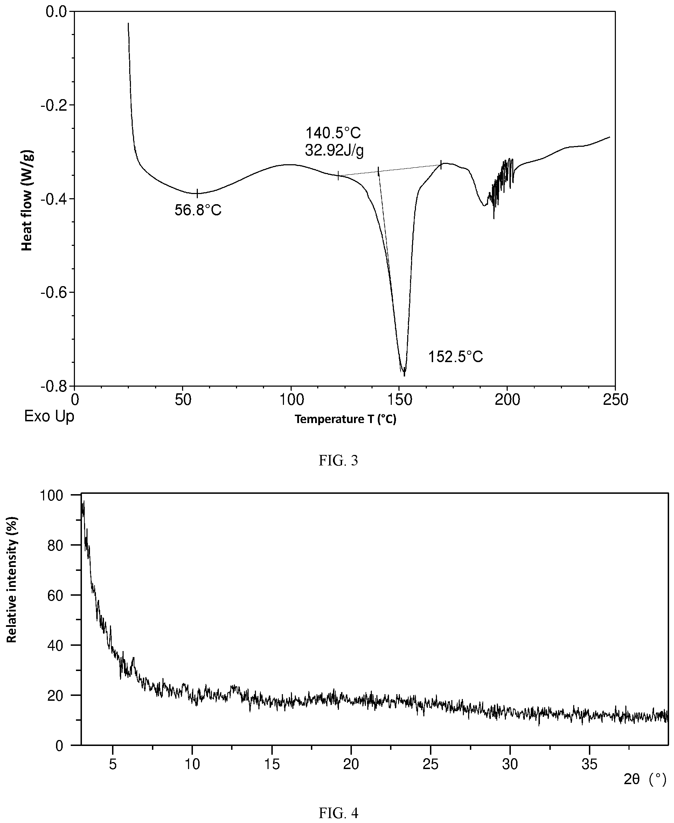

In some embodiments of the present application, the crystal form A of the compound of formula (I) has a DSC pattern as shown in FIG. 3.

In some embodiments of the present application, the crystal form A of the compound of formula (I) may exist in the form of a crystal of a solvate thereof.

The present application also provides a method for preparing the crystal form A of the compound of formula (I): comprising a step of slurrying the compound of formula (I) in acetonitrile. The method further comprises a step of separating.

In some embodiments of the present application, the crystal form A of the compound of formula (I) is prepared by the following method comprising steps of:
(1) dispersing and suspending the compound of formula (I) in acetonitrile, and slurrying;
(2) after filtration, adding the resulting solid to acetonitrile and continuing slurrying; and
(3) filtering and drying.

In some embodiments of the present application, the slurrying in the step (2) is performed under heating condition; and preferably, the slurrying is performed at temperature of 50°C.

In some embodiments of the present application, the crystal form A of the compound of formula (I) is prepared by the following method:
(1) adding methanol to a crystal form C of the compound of formula (I), suspending and stirring; and,
(2) drying.

The present application also provides a crystal form B of the compound of formula (I), wherein the crystal form B has an X-ray powder diffraction pattern comprising a diffraction peak, in terms of 2θ angle, at 12.66±0.20°.

In some embodiments of the present application, in an X-ray powder diffraction (XRPD) pattern of the crystal form B of the compound of formula (I), the peak positions and relative intensities of diffraction peaks are shown in Table 2:

**Table 2. Peak positions and relative intensities of diffraction peaks in the XRPD pattern of the crystal form B**

| No. | 2θ angle (±0.20°) | Relative intensity (%) |
|---|---|---|
| 1 | 12.66 | 10000 |

In some embodiments of the present application, the crystal form B of the compound of formula (I) has an X-ray powder diffraction pattern as shown in FIG. 4.

In some embodiments of the present application, the crystal form B of the compound of formula (I) has a thermogravimetric analysis (TGA) curve showing a weight loss of 6.24% at 150.0°C.

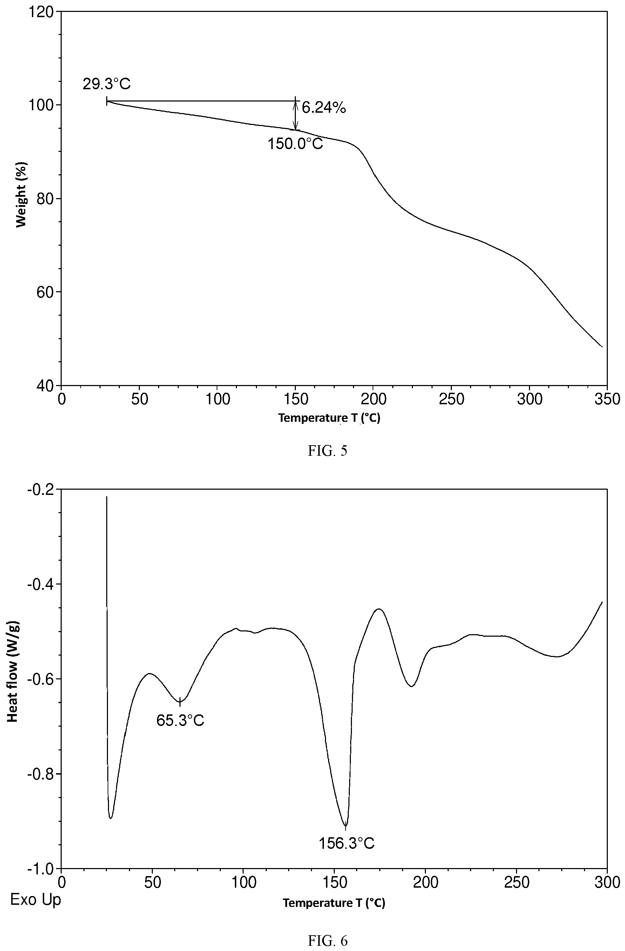

In some embodiments of the present application, the crystal form B of the compound of formula (I) has a TGA pattern as shown in FIG. 5.

In some embodiments of the present application, the crystal form B of the compound of formula (I) has a differential scanning calorimetry (DSC) curve showing endothermic peaks at 65.3°C and 156.3°C.

In some embodiments of the present application, the crystal form B of the compound of formula (I) has a DSC pattern as shown in FIG. 6.

In some embodiments of the present application, the crystal form B of the compound of formula (I) may exist in the form of a crystal of a solvate thereof.

The present application also provides a method for preparing the crystal form B of the compound of formula (I): comprising steps of suspending and stirring the compound of formula (I) in tetrahydrofuran, and then cyclically increasing and decreasing the temperature. The method further comprises a step of separating.

In some embodiments of the present application, the crystal form B of the compound of formula (I) of the present application is prepared by the following method comprising steps of:
(1) adding tetrahydrofuran to a crystal form C of the compound of formula (I), suspending and stirring;
(2) cyclically increasing and decreasing the temperature; and,
(3) drying.

The present application also provides a crystal form C of the compound of formula (I), wherein the crystal form C has an X-ray powder diffraction pattern comprising diffraction peaks, in terms of 2Θ angle, at 7.77±0.20°, 8.70±0.20°, 11.49±0.20°, 18.22±0.20°, and 23.39±0.20°.

In some embodiments of the present application, the crystal form C of the compound of formula (I) has an X-ray powder diffraction pattern comprising at least 5, 6, 7, or 8 diffraction peaks, in terms of 2θ angle, selected from the group consisting of: 7.77±0.20°, 8.70±0.20°, 11.49±0.20°, 13.45±0.20°, 18.22±0.20°, 19.82±0.20°, 21.88±0.20°, and 23.39±0.20°.

In some embodiments of the present application, the crystal form C of the compound of formula (I) has an X-ray powder diffraction pattern comprising diffraction peaks, in terms of 2Θ angle, at 7.77±0.20°, 8.70±0.20°, 11.49±0.20°, 13.45±0.20°, 18.22±0.20°, 19.82±0.20°, 21.88±0.20°, and 23.39±0.20°.

In some embodiments of the present application, the crystal form C of the compound of formula (I) has an X-ray powder diffraction pattern comprising at least 12, 13, 14, 15, or 16 diffraction peaks, in terms of 2θ angle, selected from the group consisting of: 7.77±0.20°, 8.70±0.20°, 9.97±0.20°, 10.64±0.20°, 11.49±0.20°, 13.45±0.20°, 15.55±0.20°, 18.22±0.20°, 19.82±0.20°, 20.37±0.20°, 21.88±0.20°, 23.39±0.20°, 23.99±0.20°, 27.29±0.20°, 27.75±0.20°, and 31.35±0.20°.

In some embodiments of the present application, the crystal form C of the compound of formula (I) has an X-ray powder diffraction pattern comprising diffraction peaks, in terms of 2θ angle, at 7.77±0.20°, 8.70±0.20°, 9.97±0.20°, 10.64±0.20°, 11.49±0.20°, 13.45±0.20°, 15.55±0.20°, 18.22±0.20°, 19.82±0.20°, 20.37±0.20°, 21.88±0.20°, 23.39±0.20°, 23.99±0.20°, 27.29±0.20°, 27.75±0.20°, 31.35±0.20°.

In some embodiments of the present application, the crystal form C of the compound of formula (I) has an X-ray powder diffraction pattern comprising diffraction peaks, in terms of 2θ angle, at 7.77±0.20°, 8.70±0.20°, 9.97±0.20°, 10.64±0.20°, 11.49±0.20°, 12.28±0.20°, 12.71±0.20°, 13.45±0.20°, 13.84±0.20°, 14.82±0.20°, 15.55±0.20°, 16.89±0.20°, 17.39±0.20°, 18.22±0.20°, 19.45±0.20°, 19.82±0.20°, 20.37±0.20°, 20.71±0.20°, 21.88±0.20°, 22.70±0.20°, 23.39±0.20°, 23.99±0.20°, 24.32±0.20°, 25.46±0.20°, 26.00±0.20°, 26.55±0.20°, 27.29±0.20°, 27.75±0.20°, and 31.35±0.20°.

In some embodiments of the present application, in an X-ray powder diffraction (XRPD) pattern of the crystal form C of the compound of formula (I), the peak positions and relative intensities of diffraction peaks are shown in Table 3:

**Table 3. Peak positions and relative intensities of diffraction peaks in the XRPD pattern of the crystal form C**

| No. | 2θ angle (±0.20°) | Relative intensity (%) | No. | 2θ angle (±0.20°) | Relative intensity (%) |
|---|---|---|---|---|---|
| 1 | 7.77 | 74.47 | 16 | 1982 | 42.77 |
| 2 | 8.70 | 89.14 | 17 | 20.37 | 22.44 |
| 3 | 9.97 | 15.54 | 18 | 20.71 | 18.24 |
| 4 | 10.64 | 15.60 | 19 | 21.88 | 34.24 |
| 5 | 11.49 | 75.99 | 20 | 22.70 | 10.53 |
| 6 | 12.28 | 10.50 | 21 | 23.39 | 10000 |
| 7 | 12.71 | 7.44 | 22 | 23.99 | 27.82 |
| 8 | 13.45 | 51.68 | 23 | 24.32 | 17.68 |
| 9 | 13.84 | 11.79 | 24 | 25.46 | 14.34 |
| 10 | 14.82 | 9.56 | 25 | 26.00 | 14.59 |
| 11 | 15.55 | 15.64 | 26 | 26.55 | 10.17 |
| 12 | 16.89 | 12.52 | 27 | 27.29 | 24.60 |
| 13 | 17.39 | 10.43 | 28 | 27.75 | 25.78 |
| 14 | 18.22 | 77.12 | 29 | 31.35 | 23.77 |
| 15 | 19.45 | 41.83 | | | |

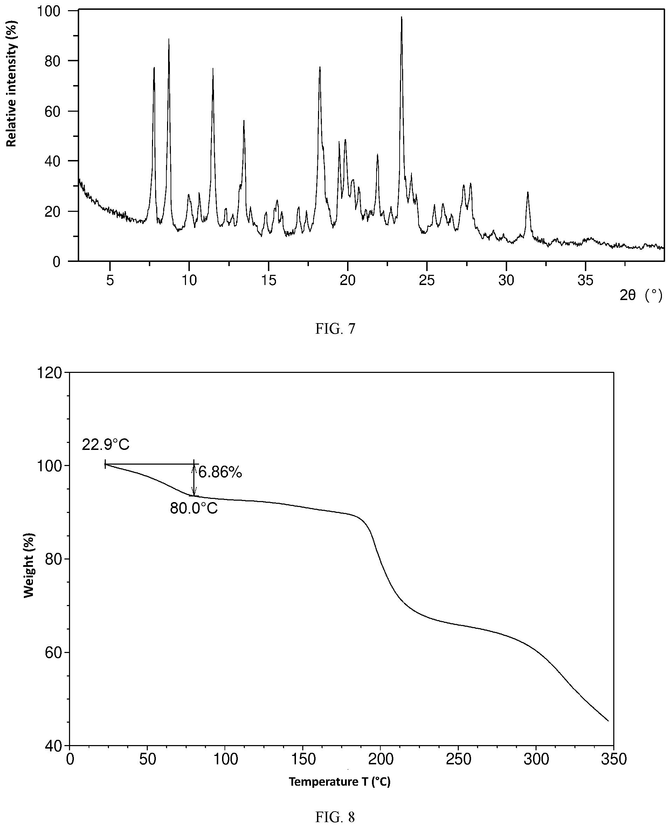

In some embodiments of the present application, the crystal form C of the compound of formula (I) has an X-ray powder diffraction pattern as shown in FIG. 7.

In some embodiments of the present application, the crystal form C of the compound of formula (I) has a thermogravimetric analysis (TGA) curve showing a weight loss of 6.86% at 80.0°C.

In some embodiments of the present application, the crystal form C of the compound of formula (I) has a TGA pattern as shown in FIG. 8.

In some embodiments of the present application, the crystal form C of the compound of formula (I) has a differential scanning calorimetry (DSC) curve showing an endothermic peak at 112.2°C.

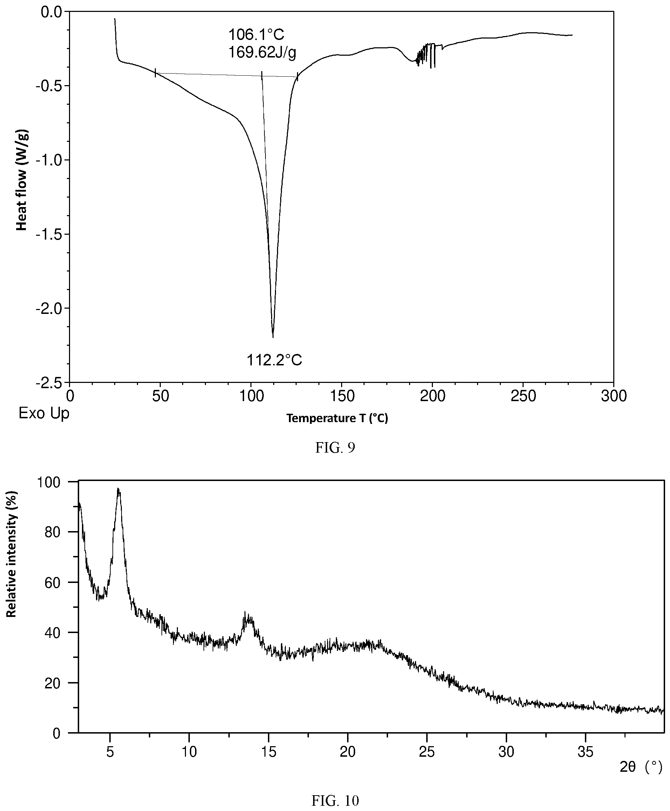

In some embodiments of the present application, the crystal form C of the compound of formula (I) has a DSC pattern as shown in FIG. 9.

In some embodiments of the present application, the crystal form C of the compound of formula (I) may exist in the form of a solvate crystal.

The present application also provides a method for preparing the crystal form C of the compound of formula (I), comprising a step of slurrying the compound of formula (I) in a mixed solvent of acetonitrile and water. The method further comprises a step of separating.

In some embodiments of the present application, in the mixed solvent of acetonitrile and water, the volume ratio of acetonitrile to water is 1:1 to 1:10.

In some embodiments of the present application, in the mixed solvent of acetonitrile and water, the volume ratio of acetonitrile to water is 1:1, 1:2, 1:3, 1:4, 1:5, 1:6, 1:7, 1:8, 1:9, 1:10 or a range formed by any of the ratios.

In some embodiments of the present application, in the mixed solvent of acetonitrile and water, the volume ratio of acetonitrile to water is 1:1 to 1:5.

In some embodiments of the present application, in the mixed solvent of acetonitrile and water, the volume ratio of acetonitrile to water is 1:3.

In some embodiments of the present application, the crystal form C of the compound of formula (I) of the present application is prepared by the following method comprising steps of:
(1) adding acetonitrile to the compound of formula (I), slurrying and stirring;
(2) adding water, slurrying and stirring;
(3) filtering and collecting the solid;
(4) washing the solid obtained in step (3) with acetonitrile; and,
(5) drying.

In some embodiments of the present application, the crystal form C of the compound of formula (I) of the present application is prepared by the following method comprising steps of:
(1) adding acetonitrile to the compound of formula (I), slurrying and stirring;
(2) evaporating about two-thirds of the acetonitrile under reduced pressure, and then adding water, slurrying and stirring;
(3) filtering and collecting the solid;
(4) adding acetonitrile to the solid obtained in step (3), slurrying and stirring; and,
(5) filtering and drying.

In some embodiments of the present application, in the step (2), the volume ratio of the remaining acetonitrile and water is 1:3.

The present application also provides a crystal form D of the compound of formula (I), wherein the crystal form D has an X-ray powder diffraction pattern comprising diffraction peaks, in terms of 2Θ angle, at 5.56±0.20° and 13.84±0.20°.

In some embodiments of the present application, in an X-ray powder diffraction (XRPD) pattern of the crystal form D, the peak positions and relative intensities of diffraction peaks are shown in Table 4:

**Table 4. Peak positions and relative intensities of diffraction peaks in the XRPD pattern of the crystal form D**

| No. | 2θ angle (±0.20°) | Relative intensity (%) |
|---|---|---|
| 1 | 5.56 | 10000 |
| 2 | 13.84 | 26.85 |

In some embodiments of the present application, the crystal form D of the compound of formula (I) has an X-ray powder diffraction pattern as shown in FIG. 10.

In some embodiments of the present application, the crystal form D of the compound of formula (I) has a thermogravimetric analysis (TGA) curve showing a weight loss of 7.96% at 150.0°C.

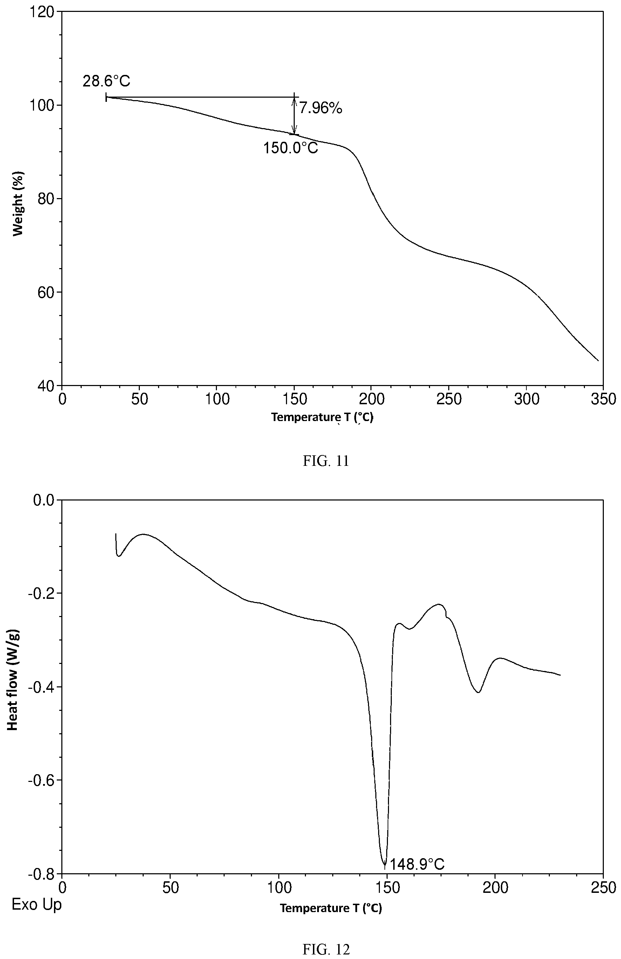

In some embodiments of the present application, the crystal form D of the compound of formula (I) has a TGA pattern as shown in FIG. 11.

In some embodiments of the present application, the crystal form D of the compound of formula (I) has a differential scanning calorimetry (DSC) curve showing an endothermic peak at 148.9°C.

In some embodiments of the present application, the crystal form D of the compound of formula (I) has a DSC pattern as shown in FIG. 12.

In some embodiments of the present application, the crystal form D of the compound of formula (I) may exist in the form of a solvate crystal.

The present application also provides a method for preparing the crystal form D of the compound of formula (I), comprising a step of suspending and stirring the crystal form C of the compound of formula (I) in tetrahydrofuran. The method further comprises a step of separating.

In some embodiments of the present application, the crystal form D of the compound of formula (I) of the present application is prepared by the following method comprising steps of:
(1) adding tetrahydrofuran to the crystal form C of the compound of formula (I), suspending and stirring; and,
(2) drying.

The present application also provides a crystal form E of the compound of formula (I), wherein the crystal form E has an X-ray powder diffraction pattern comprising diffraction peaks, in terms of 2Θ angle, at 8.53±0.20°, 9.67±0.20°, 10.31±0.20°, and 15.14±0.20°.

In some embodiments of the present application, the crystal form E of the compound of formula (I) has an X-ray powder diffraction pattern comprising diffraction peaks, in terms of 2Θ angle, at 8.53±0.20°, 9.67±0.20°, 10.31±0.20°, 15.14±0.20°, and 17.03±0.20°.

In some embodiments of the present application, the crystal form E of the compound of formula (I) has an X-ray powder diffraction pattern comprising at least 5, 6, 7, or 8 diffraction peaks, in terms of 2θ angle, selected from the group consisting of: 8.53±0.20°, 9.67±0.20°, 10.31±0.20°, 11.86±0.20°, 15.14±0.20°, 17.03±0.20°, 19.07±0.20°, and 23.60±0.20°.

In some embodiments of the present application, the crystal form E of the compound of formula (I) has an X-ray powder diffraction pattern comprising diffraction peaks, in terms of 2Θ angle, at 8.53±0.20°, 9.67±0.20°, 10.31±0.20°, 11.86±0.20°, 15.14±0.20°, 17.03±0.20°, 19.07±0.20°, and 23.60±0.20°.

In some embodiments of the present application, the crystal form E of the compound of formula (I) has an X-ray powder diffraction pattern comprising at least 10, 11, 12, or 13 diffraction peaks, in terms of 2θ angle, selected from the group consisting of: 8.53±0.20°, 9.67±0.20°, 10.31±0.20°, 11.86±0.20°, 15.14±0.20°, 16.67±0.20°, 17.03±0.20°, 18.50±0.20°, 19.07±0.20°, 21.12±0.20°, 23.60±0.20°, 26.61±0.20°, and 29.58±0.20°.

In some embodiments of the present application, the crystal form E of the compound of formula (I) has an X-ray powder diffraction pattern comprising diffraction peaks, in terms of 2Θ angle, at 8.53±0.20°, 9.67±0.20°, 10.31±0.20°, 11.86±0.20°, 15.14±0.20°, 16.67±0.20°, 17.03±0.20°, 18.50±0.20°, 19.07±0.20°, 21.12±0.20°, 23.60±0.20°, 26.61±0.20°, and 29.58±0.20°.

In some embodiments of the present application, the crystal form E of the compound of formula (I) has an X-ray powder diffraction pattern comprising diffraction peaks, in terms of 2θ angle, at 4.25±0.20°, 8.53±0.20°, 9.67±0.20°, 10.31±0.20°, 11.86±0.20°, 13.10±0.20°, 14.35±0.20°, 15.14±0.20°, 16.67±0.20°, 17.03±0.20°, 17.41±0.20°, 18.13±0.20°, 18.50±0.20°, 19.07±0.20°, 21.12±0.20°, 21.39±0.20°, 21.83±0.20°, 23.60±0.20°, 25.54±0.20°, 26.61±0.20°, 27.80±0.20°, 29.58±0.20°, and 30.59±0.20°.

In some embodiments of the present application, in an X-ray powder diffraction (XRPD) pattern of the crystal form E of the compound of formula (I), the peak positions and relative intensities of diffraction peaks are shown in Table 5:

**Table 5. Peak positions and relative intensities of diffraction peaks in the XRPD pattern of the crystal form E**

| No. | 2θ angle (±0.20°) | Relative intensity (%) | No. | 2θ angle (±0.20°) | Relative intensity (%) |
|---|---|---|---|---|---|
| 1 | 4.25 | 8.25 | 13 | 18.50 | 30.37 |
| 2 | 8.53 | 82.57 | 14 | 19.07 | 42.75 |
| 3 | 9.67 | 87.58 | 15 | 21.12 | 42.36 |
| 4 | 10.31 | 10000 | 16 | 21.39 | 29.43 |
| 5 | 11.86 | 57.15 | 17 | 21.83 | 1906 |
| 6 | 13.10 | 8.48 | 18 | 23.60 | 54.59 |
| 7 | 14.35 | 11.16 | 19 | 25.54 | 13.15 |
| 8 | 15.14 | 83.57 | 20 | 26.61 | 34.36 |
| 9 | 16.67 | 35.42 | 21 | 27.80 | 8.53 |
| 10 | 1703 | 63.34 | 22 | 29.58 | 1914 |
| 11 | 17.41 | 36.52 | 23 | 30.59 | 7.55 |
| 12 | 18.13 | 27.62 | | | |

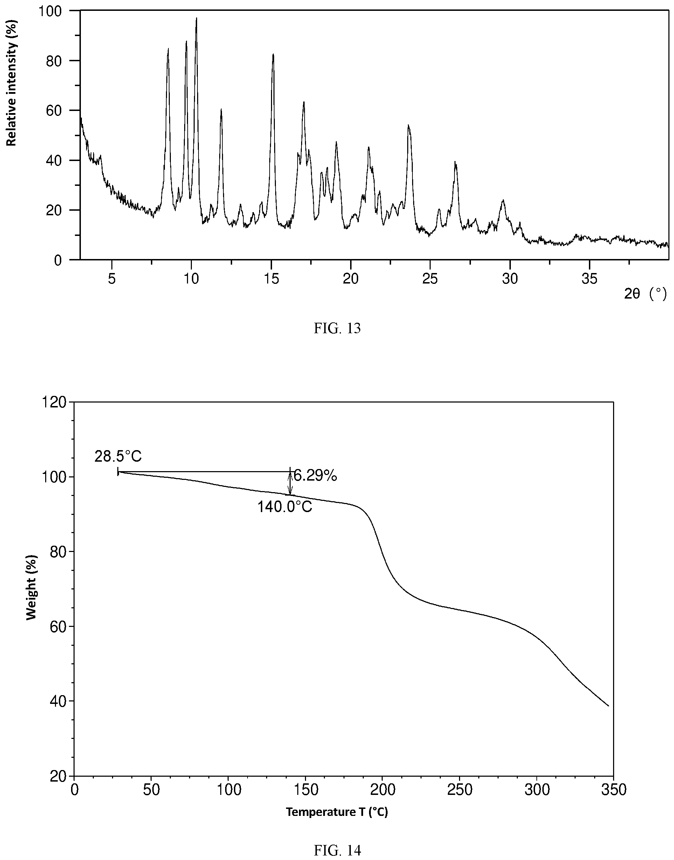

In some embodiments of the present application, the crystal form E of the compound of formula (I) has an X-ray powder diffraction pattern as shown in FIG. 13.

In some embodiments of the present application, the crystal form E of the compound of formula (I) has a thermogravimetric analysis (TGA) curve showing a weight loss of 6.29% at 140.0°C.

In some embodiments of the present application, the crystal form E of the compound of formula (I) has a TGA pattern as shown in FIG. 14.

In some embodiments of the present application, the crystal form E of the compound of formula (I) has a differential scanning calorimetry (DSC) curve showing endothermic peaks at 120.0°C, 152.7°C and 192.0°C.

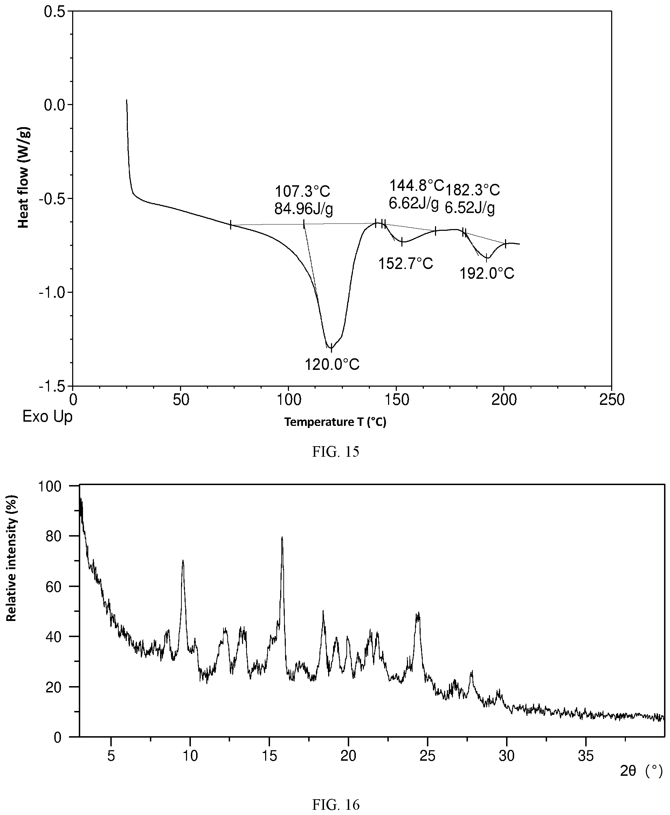

In some embodiments of the present application, the crystal form E of the compound of formula (I) has a DSC pattern as shown in FIG. 15.

In some embodiments of the present application, the crystal form E of the compound of formula (I) may exist in the form of a solvate crystal.

The present application also provides a method for preparing the crystal form E of the compound of formula (I), comprising a step of slurrying the compound of formula (I) in methanol. The method further comprises a step of separating.

In some embodiments of the present application, the crystal form E of the compound of formula (I) of the present application is prepared by the following method comprising steps of:
(1) slurrying and stirring the compound of formula (I) in methanol; and,
(2) filtering and drying.

The present application also provides a crystal form F of the compound of formula (I), wherein the crystal form F has an X-ray powder diffraction pattern comprising diffraction peaks, in terms of 2Θ angle, at 9.50±0.20°, 15.79±0.20°, and 18.44±0.20°.

The present application also provides the crystal form F of the compound of formula (I), wherein the crystal form F has an X-ray powder diffraction pattern comprising diffraction peaks, in terms of 2Θ angle, at 9.50±0.20°, 13.34±0.20°, 15.79±0.20°, 18.44±0.20°, and 24.43±0.20°.

In some embodiments of the present application, the crystal form F of the compound of formula (I) has an X-ray powder diffraction pattern comprising at least 5, 6, 7, or 8 diffraction peaks, in terms of 2θ angle, selected from the group consisting of: 9.50±0.20°, 13.34±0.20°, 15.79±0.20°, 18.44±0.20°, 19.97±0.20°, 21.27±0.20°, 21.80±0.20°, and 24.43±0.20°_{∘}

In some embodiments of the present application, the crystal form F of the compound of formula (I) has an X-ray powder diffraction pattern comprising diffraction peaks, in terms of 2Θ angle, at 9.50±0.20°, 13.34±0.20°, 15.79±0.20°, 18.44±0.20°, 19.97±0.20°, 21.27±0.20°, 21.80±0.20°, and 24.43±0.20°.

In some embodiments of the present application, in an X-ray powder diffraction (XRPD) pattern of the crystal form F of the compound of formula (I), the peak positions and relative intensities of diffraction peaks are shown in Table 6:

**Table 6. Peak positions and relative intensities of diffraction peaks in the XRPD pattern of the crystal form F**

| No. | 2θ angle (±0.20°) | Relative intensity (%) | No. | 2θ angle (±0.20°) | Relative intensity (%) |
|---|---|---|---|---|---|
| 1 | 9.50 | 75.08 | 8 | 1997 | 30.49 |
| 2 | 12.40 | 22.74 | 9 | 21.27 | 31.74 |
| 3 | 13.34 | 2985 | 10 | 21.80 | 35.38 |
| 4 | 15.79 | 100.00 | 11 | 24.43 | 55.53 |
| 5 | 16.82 | 8.22 | 12 | 27.76 | 20.97 |
| 6 | 18.44 | 40.88 | 13 | 29.52 | 10.25 |
| 7 | 19.27 | 26.54 | | | |

In some embodiments of the present application, the crystal form F of the compound of formula (I) has an X-ray powder diffraction pattern as shown in FIG. 16.

In some embodiments of the present application, the crystal form F of the compound of formula (I) has a thermogravimetric analysis (TGA) curve showing a weight loss of 2.02% at 150.0°C.

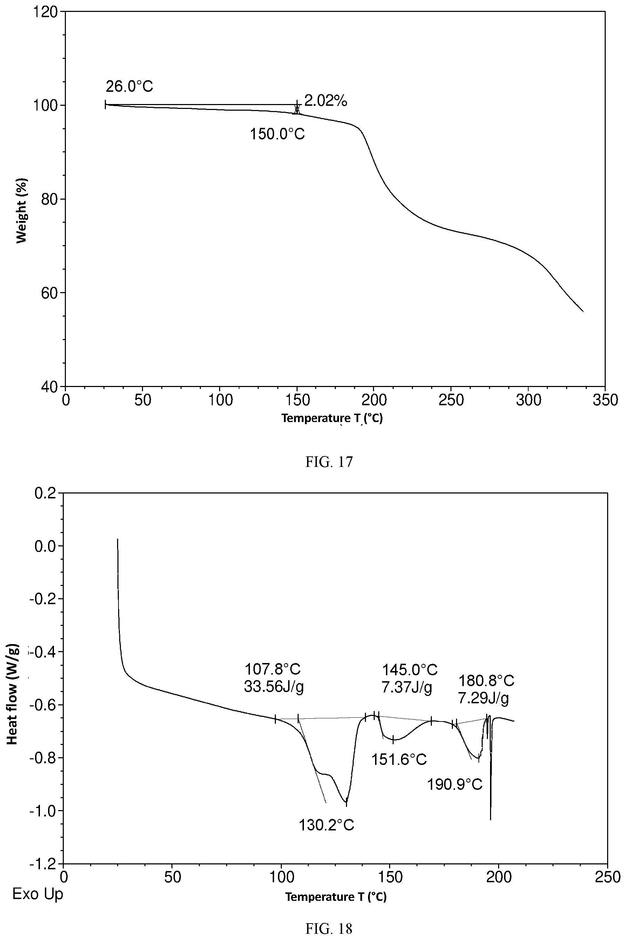

In some embodiments of the present application, the crystal form F of the compound of formula (I) has a TGA pattern as shown in FIG. 17.

In some embodiments of the present application, the crystal form F of the compound of formula (I) has a differential scanning calorimetry (DSC) curve showing endothermic peaks at 130.2°C, 151.6°C and 190.9 °C.

In some embodiments of the present application, the crystal form F of the compound of formula (I) has a DSC pattern as shown in FIG. 18.

In some embodiments of the present application, the crystal form F of the compound of formula (I) may exist in the form of a solvate crystal.

The present application also provides a method for preparing the crystal form F of the compound of formula (I), comprising a step of slurrying the crystal form E of the compound of formula (I) in methanol. The method further comprises a step of separating.

In some embodiments of the present application, the crystal form F of the compound of formula (I) of the present application is prepared by the following method comprsing the steps of:
(1) slurrying and stirring the crystal form E of the compound of formula (I) in methanol, cooling, and filtering;
(2) repeating the operation of step (1) three times; and,
(3) drying.

The present application also provides a tartrate of the compound of formula (I); preferably, the tartrate is in a crystal form.

In some embodiments of the present application, the tartrate of the compound of formula (I) is L-tartrate or D-tartrate; preferably, the L-tartrate or D-tartrate is in a crystal form.

In some embodiments of the present application, in the D-tartrate of the compound of formula (I), a molar ratio of the compound of formula (I) to D-tartaric acid is1:0.9 to 1:1.1, preferably 1:0.9, 1:1 or 1:1.1.

In some embodiments of the present application, the D-tartrate of the compound of formula (I) is the compound of formula (II), 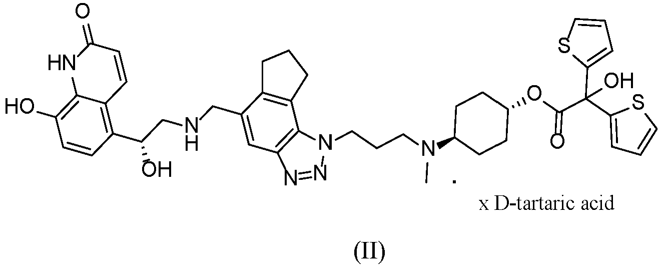 wherein, x is 0.9 to 1.1, preferably 0.9, 1 or 1.1.

In some embodiments of the present application, the compound of formula (II) is in a crystal form.

In some embodiments of the present application, the crystal of the compound of formula (II) may exist in the form of a solvate crystal.

In some embodiments of the present application, in the L-tartrate of the compound of formula (I), a molar ratio of the compound of formula (I) to L-tartaric acid is1:0.9 to 1:1.1, preferably 1:0.9, 1:1 or 1:1.1.

In some embodiments of the present application, the L-tartrate of the compound of formula (I) is the compound of formula (III), 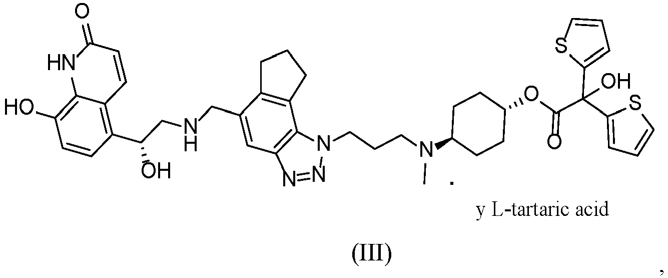 wherein, y is 0.9 to 1.1, preferably 0.9, 1 or 1.1.

In some embodiments of the present application, the compound of formula (III) is in a crystal form.

In some embodiments of the present application, the crystal of the compound of formula (III) may exist in the form of a solvate crystal.

The present application also provides a crystal form G of the compound of formula (II), wherein the crystal form G has an X-ray powder diffraction pattern comprising diffraction peaks, in terms of 2Θ angle, at 11.64±0.20°, 18.51±0.20°, and 22.68±0.20°.

In some embodiments of the present application, the crystal form G of the compound of formula (II) has an X-ray powder diffraction pattern comprising diffraction peaks, in terms of 2Θ angle, at 4.62±0.20°, 11.64±0.20°, 13.24±0.20°, 18.51±0.20°, and 22.68±0.20°.

In some embodiments of the present application, the crystal form G of the compound of formula (II) has an X-ray powder diffraction pattern comprising at least 6, 7, or 8 diffraction peaks, in terms of 2Θ angle, selected from the group consisting of: 4.62±0.20°, 10.98±0.20°, 11.64±0.20°, 13.24±0.20°, 16.76±0.20°, 18.51±0.20°, 22.68±0.20°, and 23.52±0.20°.

In some embodiments of the present application, the crystal form G of the compound of formula (II) has an X-ray powder diffraction pattern comprising diffraction peaks, in terms of 2Θ angle, at 4.62±0.20°, 10.98±0.20°, 11.64±0.20°, 13.24±0.20°, 16.76±0.20°, 18.51±0.20°, 22.68±0.20°, and 23.52±0.20°.

In some embodiments of the present application, the crystal form G of the compound of formula (II) has an X-ray powder diffraction pattern comprising at least 12, 13, 14, 15, or 16 diffraction peaks, in terms of 2θ angle, selected from the group consisting of: 4.62±0.20°, 9.80±0.20°, 10.98±0.20°, 11.64±0.20°, 12.63±0.20°, 13.24±0.20°, 13.79±0.20°, 15.28±0.20°, 16.76±0.20°, 18.51±0.20°, 19.62±0.20°, 21.62±0.20°, 22.68±0.20°, 23.52±0.20°, 25.04±0.20°, and 26.64±0.20°.

In some embodiments of the present application, the crystal form G of the compound of formula (II) has an X-ray powder diffraction pattern comprising diffraction peaks, in terms of 2Θ angle, at 4.62±0.20°, 10.98±0.20°, 11.64±0.20°, 13.24±0.20°, 13.79±0.20°, 15.28±0.20°, 16.76±0.20°, 18.51±0.20°, 19.62±0.20°, 22.68±0.20°, 23.52±0.20°, and 26.64±0.20°.

In some embodiments of the present application, the crystal form G of the compound of formula (II) has an X-ray powder diffraction pattern comprising diffraction peaks, in terms of 2Θ angle, at 4.62±0.20°, 9.80±0.20°, 10.98±0.20°, 11.64±0.20°, 12.63±0.20°, 13.24±0.20°, 13.79±0.20°, 15.28±0.20°, 16.76±0.20°, 18.51±0.20°, 19.62±0.20°, 21.62±0.20°, 22.68±0.20°, 23.52±0.20°, 25.04±0.20°, and 26.64±0.20°.

In some embodiments of the present application, the crystal form G of the compound of formula (II) has an X-ray powder diffraction pattern comprising diffraction peaks, in terms of 2θ angle, at 4.62±0.20°, 7.58±0.20°, 8.20±0.20°, 9.80±0.20°, 10.98±0.20°, 11.64±0.20°, 12.63±0.20°, 13.24±0.20°, 13.79±0.20°, 15.28±0.20°, 16.30±0.20°, 16.76±0.20°, 17.56±0.20°, 18.51±0.20°, 19.62±0.20°, 20.26±0.20°, 20.87±0.20°, 21.34±0.20°, 21.62±0.20°, 22.68±0.20°, 23.52±0.20°, 24.14±0.20°, 25.04±0.20°, 25.92±0.20°, 26.64±0.20°, 29.59±0.20°, and 30.18±0.20°. In some embodiments of the present application, in an X-ray powder diffraction (XRPD) pattern of the crystal form G of the compound of formula (II), the peak positions and relative intensities of diffraction peaks are shown in Table 7:

**Table 7. Peak positions and relative intensities of diffraction peaks in the XRPD pattern of the crystal form G**

| No. | 2θ angle (±0.20°) | Relative intensity (%) | No. | 2θ angle (±0.20°) | Relative intensity (%) |
|---|---|---|---|---|---|
| 1 | 4.62 | 40.73 | 15 | 19.62 | 25.80 |
| 2 | 7.58 | 9.02 | 16 | 20.26 | 14.11 |
| 3 | 8.20 | 5.46 | 17 | 20.87 | 10.56 |
| 4 | 9.80 | 22.62 | 18 | 21.34 | 12.38 |
| 5 | 10.98 | 46.40 | 19 | 21.62 | 15.65 |
| 6 | 11.64 | 7267 | 20 | 22.68 | 10000 |
| 7 | 12.63 | 15.41 | 21 | 23.52 | 36.90 |
| 8 | 13.24 | 52.20 | 22 | 24.14 | 10.20 |
| 9 | 13.79 | 27.79 | 23 | 25.04 | 17.66 |
| 10 | 15.28 | 35.15 | 24 | 25.92 | 1068 |
| 11 | 16.30 | 17.98 | 25 | 26.64 | 28.09 |
| 12 | 16.76 | 45.72 | 26 | 29.59 | 11.06 |
| 13 | 17.56 | 3.57 | 27 | 30.18 | 8.58 |
| 14 | 18.51 | 70.73 | | | |

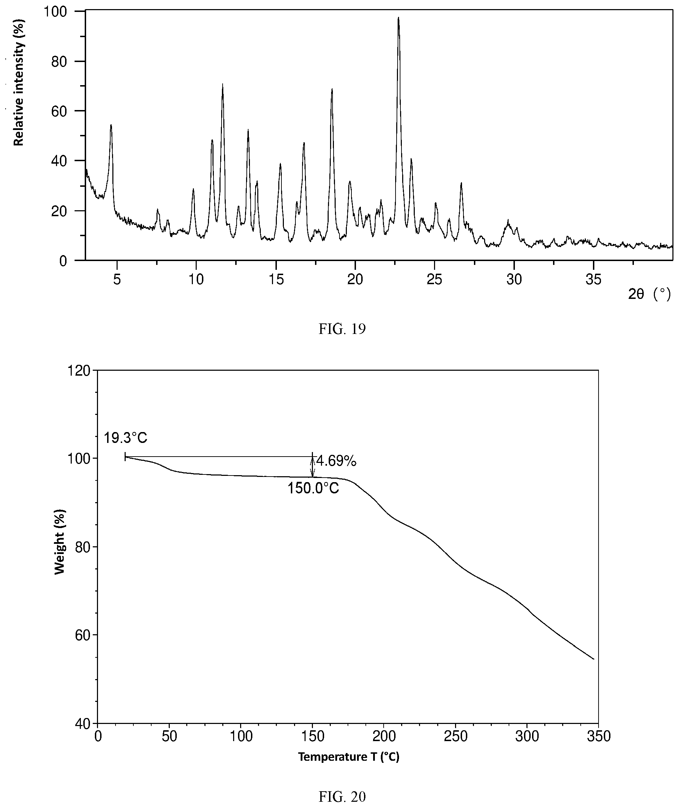

In some embodiments of the present application, the crystal form G of the compound of formula (II) has an X-ray powder diffraction pattern as shown in FIG. 19.

In some embodiments of the present application, the crystal form G of the compound of formula (II) has a thermogravimetric analysis (TGA) curve showing a weight loss of 4.69% at 150.0°C.

In some embodiments of the present application, the crystal form G of the compound of formula (II) has a TGA pattern as shown in FIG. 20.

In some embodiments of the present application, the crystal form G of the compound of formula (II) has a differential scanning calorimetry (DSC) curve showing endothermic peaks at 73.1°C, 124.5°C and 184.4°C.

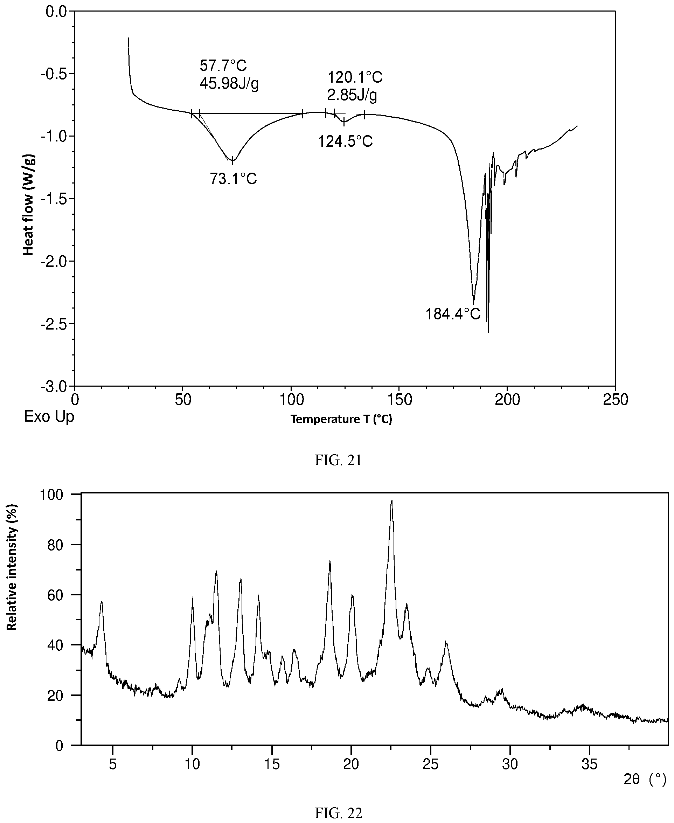

In some embodiments of the present application, the crystal form G of the compound of formula (II) has a DSC pattern as shown in FIG. 21.

In some embodiments of the present application, the crystal form G of the compound of formula (II) may exist in the form of a solvate crystal.

The present application also provides a method for preparing the crystal form G of the compound of formula (II), comprising a step of slurrying the compound of formula (II) in ethanol. The method further comprises a step of separating.

In some embodiments of the present application, the crystal form G of the compound of formula (II) of the present application is prepared by the following method comprising steps of:
(1) reacting the compound of formula (I) with D-tartaric acid in the presence of dioxane and ethanol to obtain the compound of formula (II);
(2) slurrying the compound of formula (II) obtained in step (1) in ethanol; and,
(3) filtering and drying.

The present application also provides a crystal form H of the compound of formula (III), wherein the crystal form H has an X-ray powder diffraction pattern comprising diffraction peaks, in terms of 2Θ angle, at 11.56±0.20°, 18.64±0.20°, and 22.52±0.20°.

In some embodiments of the present application, the crystal form H of the compound of formula (III) has an X-ray powder diffraction pattern comprising diffraction peaks, in terms of 2Θ angle, at 10.01±0.20°, 11.56±0.20°, 13.07±0.20°, 18.64±0.20°, and 22.52±0.20°.

In some embodiments of the present application, the crystal form H of the compound of formula (III) has an X-ray powder diffraction pattern comprising at least 5, 6, 7, or 8 diffraction peaks, in terms of 2θ angle, selected from the group consisting of: 10.01±0.20°, 11.56±0.20°, 13.07±0.20°, 14.17±0.20°, 18.64±0.20°, 20.06±0.20°, 22.52±0.20°, and 23.48±0.20°.

In some embodiments of the present application, the crystal form H of the compound of formula (III) has an X-ray powder diffraction pattern comprising diffraction peaks, in terms of 2Θ angle, at 10.01±0.20°, 11.56±0.20°, 13.07±0.20°, 14.17±0.20°, 18.64±0.20°, 20.06±0.20°, 22.52±0.20°, and 23.48±0.20°.

In some embodiments of the present application, the crystal form H of the compound of formula (III) has an X-ray powder diffraction pattern comprising at least 12, 13, 14, 15, or 16 diffraction peaks, in terms of 2θ angle, selected from the group consisting of: 4.29±0.20°, 10.01±0.20°, 10.89±0.20°, 11.56±0.20°, 13.07±0.20°, 14.17±0.20°, 14.80±0.20°, 15.66±0.20°, 16.37±0.20°, 18.64±0.20°, 20.06±0.20°, 22.52±0.20°, 23.48±0.20°, 24.80±0.20°, 25.98±0.20°, and 29.35±0.20°.

In some embodiments of the present application, the crystal form H of the compound of formula (III) has an X-ray powder diffraction pattern comprising diffraction peaks, in terms of 2Θ angle, at 4.29±0.20°, 10.01±0.20°, 10.89±0.20°, 11.56±0.20°, 13.07±0.20°, 14.17±0.20°, 16.37±0.20°, 18.64±0.20°, 20.06±0.20°, 22.52±0.20°, 23.48±0.20°, and 25.98±0.20°.

In some embodiments of the present application, the crystal form H of the compound of formula (III) has an X-ray powder diffraction pattern comprising diffraction peaks, in terms of 2Θ angle, at 4.29±0.20°, 10.01±0.20°, 10.89±0.20°, 11.56±0.20°, 13.07±0.20°, 14.17±0.20°, 14.80±0.20°, 15.66±0.20°, 16.37±0.20°, 18.64±0.20°, 20.06±0.20°, 22.52±0.20°, 23.48±0.20°, 24.80±0.20°, 25.98±0.20°, 29.35±0.20°.

In some embodiments of the present application, the crystal form H of the compound of formula (III) has an X-ray powder diffraction pattern comprising diffraction peaks, in terms of 2Θ angle, at 4.29±0.20°, 7.66±0.20°, 10.01±0.20°, 10.89±0.20°, 11.56±0.20°, 13.07±0.20°, 14.17±0.20°, 14.80±0.20°, 15.66±0.20°, 16.37±0.20°, 18.64±0.20°, 20.06±0.20°, 22.52±0.20°, 23.48±0.20°, 24.80±0.20°, 25.98±0.20°, 29.35±0.20°, and 34.55±0.20°. In some embodiments of the present application, in an X-ray powder diffraction (XRPD) pattern of the crystal form H of the compound of formula (III), the peak positions and relative intensities of diffraction peaks are shown in Table 8:

**Table 8. Peak positions and relative intensities of diffraction peaks in the XRPD pattern of the crystal form H**

| No. | 2θ angle (±0.20°) | Relative intensity (%) | No. | 2θ angle (±0.20°) | Relative intensity (%) |
|---|---|---|---|---|---|
| 1 | 4.29 | 35.58 | 10 | 16.37 | 19.14 |
| 2 | 7.66 | 3.16 | 11 | 18.64 | 67.00 |
| 3 | 10.01 | 51.17 | 12 | 20.06 | 47.25 |
| 4 | 10.89 | 35.20 | 13 | 22.52 | 10000 |
| 5 | 11.56 | 57.57 | 14 | 23.48 | 45.31 |
| 6 | 1307 | 54.28 | 15 | 24.80 | 14.12 |
| 7 | 14.17 | 47.09 | 16 | 25.98 | 29.79 |
| 8 | 14.80 | 18.22 | 17 | 29.35 | 9.17 |
| 9 | 15.66 | 15.65 | 18 | 34.55 | 5.95 |

In some embodiments of the present application, the crystal form H of the compound of formula (III) has an X-ray powder diffraction pattern as shown in FIG. 22.

In some embodiments of the present application, the crystal form H of the compound of formula (III) has a thermogravimetric analysis (TGA) curve showing a weight loss of 4.21% at 160.0°C.

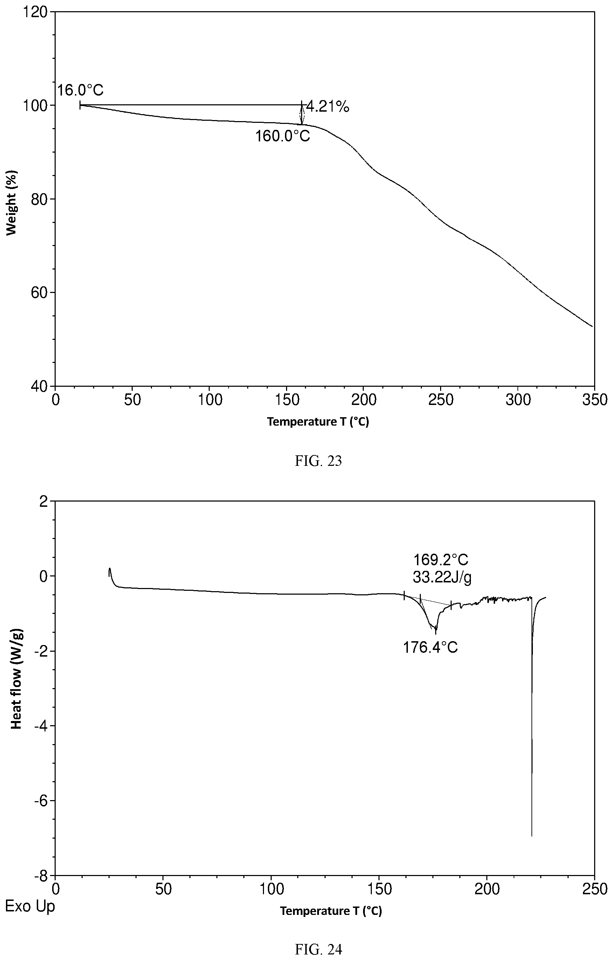

In some embodiments of the present application, the crystal form H of the compound of formula (III) has a TGA pattern as shown in FIG. 23.

In some embodiments of the present application, the crystal form H of the compound of formula (III) has a differential scanning calorimetry (DSC) curve showing an endothermic peak at 176.4°C.

In some embodiments of the present application, the crystal form H of the compound of formula (III) has a DSC pattern as shown in FIG. 24.

In some embodiments of the present application, the crystal form H of the compound of formula (III) may exist in the form of a solvate crystal.

The present application also provides a method for preparing the crystal form H of the compound of formula (III), comprising a step of precipitating the compound of formula (III) in a mixed solvent of methanol and tetrahydrofuran. The method further comprises a step of separating.

In some embodiments of the present application, the crystal form H of the compound of formula (III) of the present application is prepared by the following method comprising steps of:
(1) reacting the compound of formula (I) with L-tartaric acid in the presence of methanol and tetrahydrofuran to obtain the compound of formula (III); and,
(2) stirring, filtering and drying.

In another aspect, the present application provides a crystal composition comprising the crystal form A, the crystal form B, the crystal form C, the crystal form D, the crystal form E or the crystal form F of the compound of the formula (I), the crystal form G of the compound of the formula (II), or the crystal form H of the compound of the formula (III), wherein the crystal form makes up 50% or more, preferably 80% or more, more preferably 90% or more, and most preferably 95% or more, by weight, of the crystal composition.

In yet another aspect, the present application provides a pharmaceutical composition comprising a therapeutically effective amount of the crystal form A, the crystal form B, the crystal form C, the crystal form D, the crystal form E or the crystal form F of the compound of the formula (I), the D-tartrate of the compound of formula (I), the compound of formula (II) or the crystal form G thereof, the L-tartrate of the compound of formula (I), the compound of formula (III) or the crystal form H thereof, or the crystal composition described above. The pharmaceutical composition disclosed herein may or may not contain a pharmaceutically acceptable excipient. In addition, the pharmaceutical composition disclosed herein may further comprise one or more additional therapeutic agents.

In yet another aspect, the present application provides a method for treating chronic obstructive pulmonary disease, comprising administering to a subject in need thereof a therapeutically effective amount of the crystal form A, the crystal form B, the crystal form C, the crystal form D, the crystal form E or the crystal form F of the compound of the formula (I), the D-tartrate of the compound of formula (I), the compound of formula (II) or the crystal form G thereof, the L-tartrate of the compound of formula (I), the compound of formula (III) or the crystal form H thereof, the crystal composition described above, or the pharmaceutical composition described above.

In yet another aspect, the present application provides use of the crystal form A, the crystal form B, the crystal form C, the crystal form D, the crystal form E or the crystal form F of the compound of the formula (I), the D-tartrate of the compound of formula (I), the compound of formula (II) or the crystal form G thereof, the L-tartrate of the compound of formula (I), the compound of formula (III) or the crystal form H thereof, the crystal composition described above, or the pharmaceutical composition described above in the manufacture of a medicament for treating chronic obstructive pulmonary disease.

In yet another aspect, the present application provides use of the crystal form A, the crystal form B, the crystal form C, the crystal form D, the crystal form E or the crystal form F of the compound of the formula (I), the D-tartrate of the compound of formula (I), the compound of formula (II) or the crystal form G thereof, the L-tartrate of the compound of formula (I), the compound of formula (III) or the crystal form H thereof, the crystal composition described above, or the pharmaceutical composition described above in treating chronic obstructive pulmonary disease.

In yet another aspect, the present application provides the crystal form A, the crystal form B, the crystal form C, the crystal form D, the crystal form E or the crystal form F of the compound of the formula (I), the D-tartrate of the compound of formula (I), the compound of formula (II) or the crystal form G thereof, the L-tartrate of the compound of formula (I), the compound of formula (III) or the crystal form H thereof, the crystal composition described above, or the pharmaceutical composition described above for use in treating chronic obstructive pulmonary disease.

### Technical Effects

The compounds and the crystal forms thereof disclosed herein are easy to prepare, and have good solubility, physical stability, and chemical stability. In addition, they demonstrate good exposure via oral administration and good pharmacokinetic properties and are suitable for use as medicaments. Specifically, the crystal form C and the crystal form E of the compound of formula (I) described herein have good stability and are easy to formulate into medicines; they have significant agonistic effects on β2 receptor and binding effects on M3 receptor, and can be used as bronchodilators to treat chronic obstructive pulmonary disease.

### Definitions and Description

Unless otherwise stated, the following terms and phrases used herein are intended to have the following meanings. A particular phrase or term, unless otherwise specifically defined, should not be considered uncertain or unclear, but construed according to its common meaning. When referring to a trade name, it is intended to refer to its corresponding commercial product or its active ingredient.

For any given crystal form, the relative intensities of diffraction peaks may vary due to preferred orientations resulting from, e.g., crystal morphology, as is well known in the field of crystallography. The peak intensity varies at a place where there is preferred orientation effect, while it is impossible for the diffraction peak position of crystal form to vary. In addition, there may be slight errors in the measurement of the peak positions for any given crystal form, as is also well known in the field of crystallography. For example, the peak positions may shift due to temperature changes, sample movement, or calibration of the instrument when analyzing a sample, and the error in the measurement of 2Θ is sometimes about ±0.2 degree, and therefore, it is well known to those skilled in the art that this error should be taken into account when determining each crystal structure.

DSC measures the transition temperature when a crystal form absorbs or releases heat due to a change in crystal structure or melting of the crystal form. For the same crystal forms of the same compound, the thermal transition temperature and melting point errors in successive analyses are typically within about 5°C or 3°C, and a given DSC peak or melting point of a compound, when referred to, means the DSC peak or melting point ± 5°C or ± 3°C. DSC provides an auxiliary method to identify different crystal forms. Different crystal morphologies can be identified by their different transition temperatures. It should be noted that for a mixture, its DSC peak or melting point may vary over a larger range. Furthermore, the melting temperature is related to the heating rate due to decomposition in the melting process of a substance.

For the same crystal form, the temperature of a weight loss in the TGA may vary due to measuring instrument, measuring method/condition, and other factors. For any given crystal form, the temperature of a weight loss may have an error of about ±5 °C or about ±3 °C.

It should be noted that during preparation of a crystal form of a drug, when the drug molecules and the solvent molecules are in contact with each other, it is difficult to avoid that the solvent molecules with the compound molecules form eutectics and remain in the solid due to external conditions and internal factors, thereby forming a solvate, specifically including a stoichiometric and non-stoichiometric solvate. Such solvates are encompassed within the scope of the present invention.

The "pharmaceutically acceptable excipient" refers to an inert substance administered with an active ingredient to facilitate administration of the active ingredient, including but not limited to, any glidant, sweetener, diluent, preservative, dye/coloring agent, flavor enhancer, surfactant, wetting agent, dispersant, disintegrant, suspending agent, stabilizer, isotonizing agent, solvent or emulsifier acceptable for use in humans or animals (e.g., domesticated animals) as permitted by the National Medical Products Administration, PRC.

The term "crystal composition" refers to a mixture consisting of one or more of the crystal forms of the compound of formula (I), or formula (II), or formula (III) disclosed herein and other crystal forms or amorphous forms of the compound, or other impurities. For example, a crystal composition of the crystal form A of the compound of formula (I) refers to a mixture comprising, in addition to the crystal form A of the compound of formula (I), other crystal forms or amorphous forms of the compound of formula (I), or other impurities.

The term "pharmaceutical composition" refers to a mixture consisting of one or more of the compounds or the salts thereof disclosed herein, and a pharmaceutically acceptable excipient. The pharmaceutical composition is intended to facilitate the administration of the compound of the present application to an organic entity.

Therapeutic dosages of the compound of the present application may be determined by, for example, the specific use of a treatment, the route of administration of the compound, the health and condition of a patient, and the judgment of a prescribing physician. The proportion or concentration of the compound of the present application in a pharmaceutical composition may not be constant and depends on a variety of factors including dosages, chemical properties (e.g., hydrophobicity), and routes of administration.

The term "treating" or "treatment" means administering the compound or formulation described herein to ameliorate or eliminate a disease or one or more symptoms associated with the disease, and includes:
(i) inhibiting a disease or disease state, i.e., arresting its progression; and
(ii) alleviating a disease or disease state, i.e., causing its regression.

The term "therapeutically effective amount" refers to an amount of the compound disclosed herein for (i) treating a specific disease, condition or disorder; (ii) alleviating, relieving or eliminating one or more symptoms of a specific disease, condition or disorder, or (iii) preventing or delaying onset of one or more symptoms of a specific disease, condition or disorder described herein. The amount of the compound disclosed herein that is considered as the "therapeutically effective amount" varies depending on the compound, the disease state and its severity, the administration regimen, and the age of the mammal to be treated, but can be determined routinely by those skilled in the art in accordance with their knowledge and the present disclosure.

Unless otherwise required, the word "comprise" and variations thereof such as "comprises" and "comprising" and equivalents thereof, used in the specification and the claims which follow, should be understood in an open-ended and non-exclusive sense, i.e., "including, but not limited to".

"One embodiment", "an embodiment", "in another embodiment" or "in some embodiments" used in the specification means that a specific reference element, structure or feature described in connection with the embodiment is included in at least one embodiment. Thus, the phrases "in one embodiment", "in an embodiment", "in another embodiment" and "in some embodiments" in various places throughout the specification are not necessarily all referring to the same embodiment. Furthermore, the specific elements, structures, or features may be combined in any suitable manner in one or more embodiments.

It should be understood that, unless otherwise specified clearly, the singular forms "a", "an", and "the" used in the specification and the appended claims of the present application include plural referents. Thus, for example, the mentioned reaction including "a catalyst" includes one catalyst, or two or more catalysts. It should be understood that, unless otherwise specified clearly, the term "or" is generally used in its sense including "and/or".

The intermediate compounds disclosed herein can be prepared by a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples disclosed herein.

The chemical reactions of the embodiments of the present application are carried out in a proper solvent that should be suitable for the chemical changes in the present application and the reagents and materials required. In order to acquire the compounds of the present application, it is sometimes necessary for those skilled in the art to modify or select a synthesis procedure or a reaction scheme based on the existing embodiments.

The configuration of the 1,4-cyclohexyl group in the compound structures of the present application is all *trans*; for example, the compound 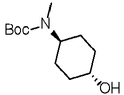 represents *trans*-(4-hydroxy-cyclohexyl)-methylcarbamic acid *tert*-butyl ester; for another example, the configuration of the cyclohexyl group in the compound of formula (I) disclosed herein is *trans.*

The present application is described in detail below by way of examples, which, however, are not intended to disadvantageously limit the scope of the present application in any way.

All solvents used in the present application are commercially available and can be used without further purification. The solvents used in the present application are commercially available.

The following abbreviations are used in the present application: Boc represents tert-butyloxycarbonyl; THF represents tetrahydrofuran; DMSO represents dimethyl sulfoxide; BoczO represents di-tert-butyl dicarbonate; Bn represents benzyl; Cbz represents benzyloxycarbonyl; TBS represents tert-butyldimethylsilyl; DIEA represents N,N-diisopropylethylamine; DMF represents N,N-dimethylformamide; i-PrOAc represents isopropyl acetate; DIPEA represents N,N-diisopropylethylamine; HEPES represents 4-hydroxyethylpiperazine ethanesulfonic acid; NMS represents N-methyl-scopolamine; PEI represents polyethyleneimine; CbzCl represents benzyl chloroformate; DCM represents dichloromethane; EA or EtOAc represents ethyl acetate; PE represents petroleum ether; MeOH represents methanol; Ru-(S,S)-Ms-DENEB represents {N-[(1S,2S)-2-[(R)-[2-[[1,2,3,4,5,6-η)-4-methylphenyl] methoxy]ethyl]amino]-1,2-diphenylethylmethanesulfonamido}ruthenium(II) chloride; Xphos represents 2-dicyclohexylphosphino-2,4,6-triisopropylbiphenyl; Pd₂(dba)₃ represents bis(dibenzylideneacetone)palladium; MsCl represents methanesulfonyl chloride; DIBAL-H represents diisobutylaluminum hydride.

The compounds disclosed herein are named according to conventional nomenclature rules in the art or using ChemDraw^{®} software, and supplier's catalog names are given for commercially available compounds.

### Instruments and analytical methods

### 1.1. X-ray powder diffractometer (XRPD) method of the present application

Instrument model: X' Pert3.
Method: taking a sample of about 10 to 20 mg for XRPD analysis.

Detailed XRPD parameters are as follows:
X-ray source: Cu, kα (Kα1=1.540598 Å, Kα2=1.544426 Å, Kα2/Kα1 intensity ratio: 0.5)
Tube voltage: 45 kV, tube current: 40 mA
Divergence slit: fixed 1/8°
First seller slit: 0.04 rad, Second seller slit: 0.04 rad
Receiving slit: none, Anti-scatter slit: 7.5 mm
Measurement time: 5 min
scan range: 3 to 40°
Step width angle: 0.0263°
Step length: 46.665 seconds
Rotation speed of sample plate: 60 rpm

### 1.2 Differential Scanning Calorimeter (DSC) method of the present application

Instrument model: TA Q200/Q2000/2500 Differential Scanning Calorimeter
Test method: Taking a sample (about 1 to 5 mg) and placing in a DSC aluminum dish for testing, and heating the sample from 25°C (room temperature) to a temperature before decomposition of the sample at a heating rate of 10°C/min under 50 mL/min N₂ conditions.

### 1.3 Thermal Gravimetric Analyzer (TGA) method of the present application

Instrument model: TA Q5000/5500 Thermal Gravimetric Analyzer
Test method: Taking a sample (about 1 to 5 mg) and placing in a TGA aluminum dish for testing, and heating the sample from room temperature to 350°C at a heating rate of 10°C/min under 10 mL/min N₂ conditions.

### 1.4 Dynamic Vapor Sorption (DVS) method of the present application

Instrument model: SMS DVS Advantage Dynamic Vapor Sorption Analyzer
Test conditions: Taking a sample (10 to 15 mg) and placing in a DVS sample dish for testing.

Detailed DVS parameters are as follows:
Temperature: 25°C
Equilibration: dm/dt=0.01%/min (Minimum: 10 min, Maximum: 180 min)
Drying: drying 120 min under 0% RH
RH (%) test gradient: 10%
RH (%) test gradient range: 0% to 90% to 0%.

### BRIEF DESCRIPTION OF THE DRAWINGS

FIG. 1 is an XRPD pattern of the crystal form A of the compound of Formula (I) obtained using Cu-Kα radiation.
FIG. 2 is a TGA pattern of the crystal form A of the compound of Formula (I).
FIG. 3 is a DSC pattern of the crystal form A of the compound of Formula (I).
FIG. 4 is an XRPD pattern of the crystal form B of the compound of Formula (I) obtained using Cu-Kα radiation.
FIG. 5 is a TGA pattern of the crystal form B of the compound of Formula (I).
FIG. 6 is a DSC pattern of the crystal form B of the compound of Formula (I).
FIG. 7 is an XRPD pattern of the crystal form C of the compound of Formula (I) obtained using Cu-Kα radiation.
FIG. 8 is a TGA pattern of the crystal form C of the compound of Formula (I).
FIG. 9 is a DSC pattern of the crystal form C of the compound of Formula (I).
FIG. 10 is an XRPD pattern of the crystal form D of the compound of Formula (I) obtained using Cu-Kα radiation.
FIG. 11 is a TGA pattern of the crystal form D of the compound of Formula (I).
FIG. 12 is a DSC pattern of the crystal form D of the compound of Formula (I).
FIG. 13 is an XRPD pattern of the crystal form E of the compound of Formula (I) obtained using Cu-Kα radiation.
FIG. 14 is a TGA pattern of the crystal form E of the compound of Formula (I).
FIG. 15 is a DSC pattern of the crystal form E of the compound of Formula (I).
FIG. 16 is an XRPD pattern of the crystal form F of the compound of Formula (I) obtained using Cu-Kα radiation.
FIG. 17 is a TGA pattern of the crystal form F of the compound of Formula (I).
FIG. 18 is a DSC pattern of the crystal form F of the compound of Formula (I).
FIG. 19 is an XRPD pattern of the crystal form G of the compound of Formula (II) obtained using Cu-Kα radiation.
FIG. 20 is a TGA pattern of the crystal form G of the compound of Formula (II).
FIG. 21 is a DSC pattern of the crystal form G of the compound of Formula (II).
FIG. 22 is an XRPD pattern of the crystal form H of the compound of Formula (III) obtained using Cu-Kα radiation.
FIG. 23 is a TGA pattern of the crystal form H of the compound of Formula (III).
FIG. 24 is a DSC pattern of the crystal form H of the compound of Formula (III).
FIG. 25 is a DVS pattern of the crystal form C of the compound of Formula (I).
FIG. 26 shows an XRPD pattern (obtained using Cu-Kα) overlay of the crystal form C of the compound of Formula (I) before and after DVS test.
FIG. 27 shows an XRPD pattern (obtained using Cu-Kα) overlay of the crystal form E of the compound of Formula (I) before and after storaging under 94%RH.

### DETAILED DESCRIPTION

The present application is described in detail below by way of examples, which, however, are not intended to disadvantageously limit the scope of the present application in any way. The present application has been described in detail herein, wherein specific embodiments thereof are also disclosed. It will be apparent to those skilled in the art that various changes and improvements can be made to the specific embodiments of the present application without departing from the spirit and scope of the present application.

### Reference Example 1

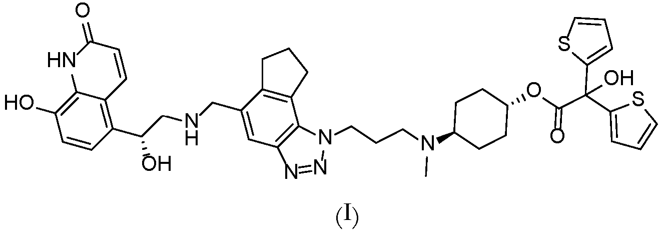

### Synthetic route:

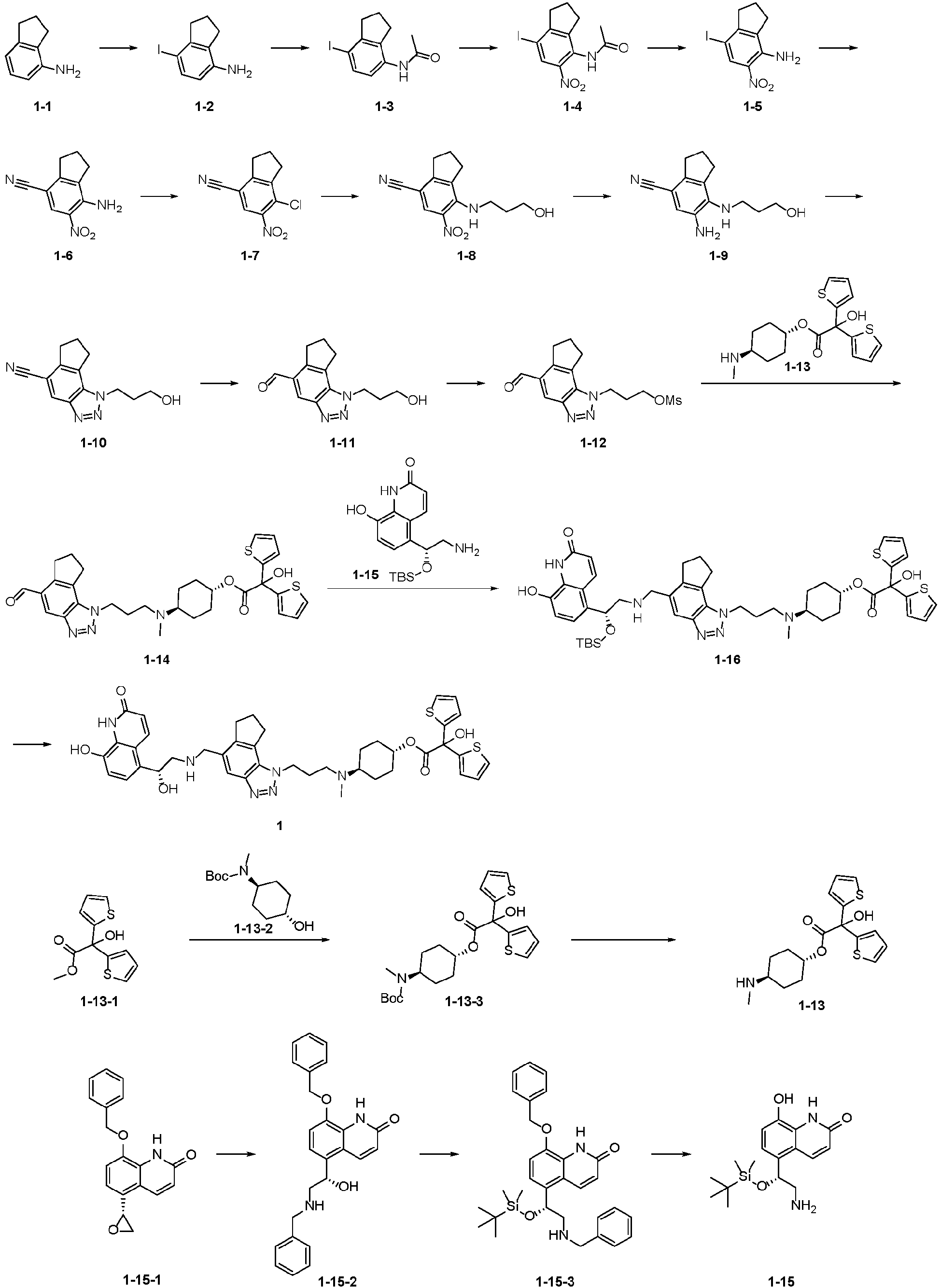

### Step 1: synthesis of compound 1-2

Compound **1-1** (15 g) was dissolved in acetic acid (75 mL) and water (75 mL), and sodium perborate (17.33 g) was added under stirring. The mixture was cooled to 0 °C and a solution of potassium iodide (18.70 g) in water (150 mL) was slowly added dropwise. The mixture was heated to room temperature (25 °C) and stirred for 0.5 h. After the reaction was completed, the reaction solution was filtered, and the filter cake was washed with 500 mL of water. The solid was collected and concentrated to dryness under reduced pressure to give compound **1-2,** which was directly used in the next step.

¹H NMR (400MHz, CDCl₃) δ: 7.32 (d, *J*=8.4 Hz, 1H), 6.30 (d, *J*=8.4 Hz, 1H), 3.65 (s, 2H), 2.92 - 2.84 (m, 4H), 2.18 - 2.05 (m, 2H).

MS-ESI calcd. [M+H]⁺ 260, found 260.0.

### Step 2: synthesis of compound 1-3

Compound **1-2** (26.5 g) was dissolved in tetrahydrofuran (250 mL), and acetic anhydride (15.66 g) and diisopropylethylamine (26.44 g) were added. The mixture was heated to 45 °C and stirred for 16 h. After the reaction was completed, the reaction solution was diluted with 500 mL of ethyl acetate, washed with 500 mL of water and 500 mL of saturated brine sequentially, dried over anhydrous sodium sulfate, filtered, and concentrated to dryness under reduced pressure to give compound **1-3,** which was directly used in the next step.

MS-ESI calcd. [M+H]⁺ 302, found 302.0.

### Step 3: synthesis of compound 1-4

To a suspension of compound **1-3** (5 g) in acetic anhydride (50 mL) was added concentrated nitric acid (2.41 g, purity: 65%) at 0 °C. The mixture was stirred at 0-25 °C for 2 h. After the reaction was completed, the reaction solution was filtered, and the filter cake was washed with 50 mL of petroleum ether. The solid was collected and concentrated to dryness under reduced pressure to give compound **1-4,** which was directly used in the next step.

¹H NMR (400MHz, CDCl₃) δ: 8.69 (s, 1H), 8.26 (s, 1H), 3.06 - 2.97 (m, 4H), 2.26 - 2.21 (m, 3H), 2.14 (t, *J*=7.6 Hz, 2H).

MS-ESI calcd. [M+H]⁺ 347, found 346.9.

### Step 4: synthesis of compound 1-5

Compound **1-4** (14.5 g) was dissolved in ethanol (400 mL), and hydrochloric acid solution (6 M, 139.64 mL) was added. The mixture was heated to 85 °C and stirred for 72 h. After the reaction was completed, the reaction solution was concentrated under reduced pressure to remove most of ethanol and filtered. The precipitated solid was collected and concentrated to dryness reduced pressure to give compound **1-5,** which was directly used in the next step.

¹H NMR (400MHz, CDCl₃) δ: 8.38 (s, 1H), 5.96 (s, 2H), 3.03 - 2.83 (m, 4H), 2.28 - 2.09 (m, 2H).

MS-ESI calcd. [M+H]⁺ 305, found 304.9.

### Step 5: synthesis of compound 1-6

Compound **1-5** (8.5 g) was dissolved in dimethyl sulfoxide (100 mL), and cuprous oxide (799.95 mg) and acetyl nitrile (3.86 g) were added. The mixture was heated to 130 °C and stirred for 4 h. After the reaction was completed, the reaction solution was cooled to room temperature and poured into 750 mL of water, and the resulting mixture was extracted with ethyl acetate (350 mL × 2). The organic phases were combined, washed with saturated brine (350 mL × 1), dried over anhydrous sodium sulfate, filtered and concentrated under reduced pressure, and the residue was separated and purified by flash silica gel column chromatography (eluent: ethyl acetate/petroleum ether, 0-30%) to give compound **1-6.**

¹H NMR (400MHz, CDCl₃) δ: 8.38 (s, 1H), 6.37 (s, 2H), 3.15 (t, *J*=7.6 Hz, 2H), 2.84 (t, *J*=7.6 Hz, 2H), 2.35 - 2.26 (m, 2H).

### Step 6: synthesis of compound 1-7

A mixture of cuprous chloride (6.25 g) and acetonitrile (470 mL) was heated to 65 °C, *tert*-butyl nitrite (5.99 g) was added in one portion, and compound **1-6** (4.72 g) was added in portions. The mixture was stirred at 65 °C for 0.5 h. After the reaction was completed, the reaction solution was cooled to room temperature and concentrated to dryness under reduced pressure. Ethyl acetate (500 mL) and hydrochloric acid solution (6 M, 200 mL) were added to the residue, followed by liquid separation. The organic phase was washed with saturated brine (500 mL), dried over anhydrous sodium sulfate, filtered and concentrated under reduced pressure, and the residue was separated and purified by flash silica gel column chromatography (eluent: ethyl acetate/petroleum ether, 0-10%) to give compound **1-7.**

¹H NMR (400MHz, CDCl₃) δ: 8.01 (s, 1H), 3.28 (t, *J*=7.6 Hz, 2H), 3.16 (t, *J*=7.6 Hz, 2H), 2.31 (t, *J*=7.6 Hz, 2H).

### Step 7: synthesis of compound 1-8

Compound **1-7** (3.74 g) was dissolved in tetrahydrofuran (150 mL), and triethylamine (4.25 g) and 3-aminopropanol (3.79 g) were added. The mixture was heated to 65 °C and stirred for 16 h. After the reaction was completed, the reaction solution was cooled to room temperature and poured into 300 mL of water, and the resulting mixture was extracted with ethyl acetate (300 mL). The organic phase was washed with saturated brine (300 mL × 1), dried over anhydrous sodium sulfate, filtered and concentrated under reduced pressure, and the residue was separated and purified by flash silica gel column chromatography (eluent: dichloromethane, 100%) to give compound **1-8.**

¹H NMR (400MHz, CDCl₃) δ: 8.66 (s, 1H), 8.37 (s, 1H), 3.90 - 3.75 (m, 4H), 3.29 (t, *J*=7.2 Hz, 2H), 3.04 (t, *J*=7.6 Hz, 2H), 2.23 - 2.13 (m, 2H), 1.96 - 1.88 (m, 2H), 1.47 (t, *J*=4.4 Hz, 1H).

MS-ESI calcd. [M+H]⁺ 262, found 261.9.

### Step 8: synthesis of compound 1-9

Compound **1-8** (2.35 g) was dissolved in a mixed solution of methanol, tetrahydrofuran and water (135 mL, volume ratio: 1/1/1), and reduced iron powder (3.01 g) and ammonium chloride (2.41 g) were added. The mixture was heated to 70 °C and stirred for 2 h. After the reaction was completed, the reaction solution was filtered, and the filtrate was collected, concentrated to dryness under reduced pressure, and separated and purified by flash silica gel column chromatography (eluent: ethyl acetate/petroleum ether, 0-50%) to give compound **1-9.**

¹H NMR (400MHz, CDCl₃) δ: 6.79 (s, 1H), 3.91 - 3.81 (m, 2H), 3.41 (s, 2H), 3.05 - 2.92 (m, 4H), 2.16 - 2.08 (m, 2H), 1.88 - 1.79 (m, 2H).

MS-ESI calcd. [M+H]⁺ 232, found 232.0.

### Step 9: synthesis of compound 1-10

Compound **1-9** (2 g) was dispersed in hydrochloric acid solution (6 M, 14.41 mL), and the system was cooled to 0 °C. A solution of sodium nitrite (894.97 mg) in water (8 mL) was slowly added dropwise at 0 °C. The mixture was stirred at 0-25 °C for 1 h. After the reaction was completed, the reaction solution was subjected to liquid separation in ethyl acetate (200 mL) and water (200 mL). The organic phase was washed with saturated brine (100 mL), dried over anhydrous sodium sulfate, filtered and concentrated to dryness under reduced pressure to give compound **1-10,** which was directly used in the next step.

MS-ESI calcd. [M+H]⁺ 243, found 243.0.

### Step 10: synthesis of compound 1-11

Compound **1-10** (2 g) was dissolved in formic acid solution (80 mL, purity: 75%), and nickel-aluminum alloy (3.54 g) was added. The mixture was heated to 90 °C and stirred for 16 h. The reaction solution was filtered, and the filtrate was collected and concentrated to dryness under reduced pressure. 200 mL of ethanol was added, and an aqueous sodium hydroxide solution (2 M, 41.28 mL) was added under stirring. The mixture was stirred at 25 °C for 1 h. After the reaction was completed, the reaction solution was cooled in an ice bath, adjusted to pH 6-7 with 2 N hydrochloric acid solution, concentrated under reduced pressure to remove most of methanol, and extracted with ethyl acetate (250 mL × 2). The organic phase was washed with saturated brine (200 mL), dried over anhydrous sodium sulfate, filtered and concentrated to dryness under reduced pressure to give compound **1-11,** which was directly used in the next step.

MS-ESI calcd. [M+H]⁺ 246, found 246.0.

### Step 11: synthesis of compound 1-12

Compound **1-11** (250 mg) was dissolved in dichloromethane (20 mL), and triethylamine (309.41 mg) and methanesulfonyl chloride (233.51 mg) were added. The mixture was stirred at 25 °C for 0.5 h. Two reactions were set up in parallel. After the reaction was completed, the reaction solution was subjected to liquid separation in dichloromethane (200 mL) and water (200 mL). The organic phase was washed with saturated brine (100 mL), dried over anhydrous sodium sulfate, filtered, concentrated to dryness under reduced pressure, and separated and purified by flash silica gel column chromatography (eluent: methanol/dichloromethane, 0-2%) to give compound **1-12.** MS-ESI calcd. [M+H]⁺ 324, found 324.0.

### Step 12: synthesis of compound 1-13-3

Compound **1-13-1** (11.09 g) dissolved in anhydrous toluene (100 mL) was added to compound **1-13-2** (10 mg) dissolved in anhydrous toluene (100 mL), followed by the addition of sodium hydride (872 mg, purity: 60%). The mixture was stirred at 155 °C for 2 h. The reaction solution was cooled to room temperature, and the reaction was quenched with 4% aqueous sodium bicarbonate solution (200 mL). The resulting mixture was extracted with ethyl acetate (200 mL × 2). The organic phases were combined, washed with saturated brine (200 mL), dried over anhydrous sodium sulfate, filtered and concentrated under reduced pressure, and the residue was separated and purified by silica gel column chromatography (petroleum ether/ethyl acetate, 20:1-6:1) to give compound **1-13-3.** MS-ESI calcd. [M+H-18]⁺ 434, found 434.1.

### Step 13: synthesis of compound 1-13

Compound **1-13-3** (400 mg) was dissolved in ethyl acetate (4 mL), and a solution of hydrogen chloride in ethyl acetate (6.64 mL, 4 M) was added. The mixture was stirred at 25 °C for 2 h. The reaction solution was adjusted to about pH 8 with a saturated aqueous sodium carbonate solution and extracted with ethyl acetate (30 mL × 3). The organic phases were combined, washed with saturated brine (100 mL), dried over anhydrous sodium sulfate, filtered and concentrated under reduced pressure to give crude compound **1-13.**

MS-ESI calcd. [M+H]⁺ 352, found 352.1.

### Step 14: synthesis of compound 1-14

Compound **1-13** (200 mg) was dissolved in acetonitrile (20 mL), and diisopropylethylamine (220.62 mg), potassium iodide (141.69 mg) and compound **1-12** (184.00 mg) were added. The mixture was heated to 90 °C and stirred for 16 h. After the reaction was completed, the reaction solution was concentrated to dryness under reduced pressure, and separated and purified by flash silica gel column chromatography (eluent: methanol/dichloromethane, 0-5%) to give compound **1-14.**

MS-ESI calcd. [M+H]⁺ 579, found 579.2.

### Step 15: synthesis of acetate salt of compound 1-15

Compound **1-15-1** (2 g) was added to benzylamine (4.92 g). The mixture was stirred at 150 °C for 0.5 h under microwave irradiation. The reaction was quenched with water (40 mL), and the resulting mixture was extracted with ethyl acetate (40 mL × 3). The organic phases were combined, washed with saturated brine (80 mL), dried over anhydrous sodium sulfate, filtered and concentrated under reduced pressure, and the residue was separated and purified by silica gel column chromatography (petroleum ether/ethyl acetate, 10:1-0:1, dichloromethane/methanol: 20:1-10: 1) to give compound **1-15-2.** MS-ESI calcd. [M+H]⁺ 401, found 401.1.

Compound **1-15-2** (50 mg) was dissolved in DCM (1 mL), and 2,6-dimethylpyridine (26.8 mg) and *tert-*butyldimethylsilyl trifluoromethanesulfonate (36.30 mg) were added at 0 °C. The mixture was stirred at 20 °C for 2 h. The reaction was quenched with water (2 mL), and the resulting mixture was extracted with dichloromethane (2 mL × 2). The organic phases were combined, washed with saturated brine (2 mL), dried over anhydrous sodium sulfate, filtered and concentrated under reduced pressure to give crude compound **1-15-3.** MS-ESI calcd. [M+H]⁺ 515, found 515.2.

Compound **1-15-3** (10 g) was dissolved in methanol (100 mL), and acetic acid (1.67 mL) and Pd(OH)₂ (2 g, 20%) were added. The mixture was stirred at 20 °C for 16 h under hydrogen atmosphere (15 psi). The reaction solution was filtered and concentrated under reduced pressure to give an acetate salt of crude compound **1-15.** MS-ESI calcd. [M+H]⁺ 335, found 334.9.

### Step 16: synthesis of compound 1-16

Compound **1-14** (325 mg) and compound **1-15** (187.83 mg) were dissolved in methanol (10 mL) and tetrahydrofuran (8 mL), and 4A molecular sieve (300 mg), diisopropylethylamine (217.73 mg) and sodium triacetoxyborohydride (595.09 mg) were added. The mixture was stirred at 25 °C for 3 h, and sodium triacetoxyborohydride (595.09 mg) was added. The mixture was stirred at 25 °C for 16 h, and sodium triacetoxyborohydride (595.09 mg) was added. The mixture was stirred at 25 °C for 3 h. The reaction solution was concentrated to dryness, and 10 mL of water was added. The resulting mixture was extracted with dichloromethane (20 mL × 2). The organic phase was washed with saturated brine (20 mL), dried over anhydrous sodium sulfate, filtered, concentrated to dryness under reduced pressure, and separated and purified by flash silica gel column chromatography (eluent: methanol/dichloromethane, 0-10%) to give compound **1-16.**

MS-ESI calcd. [M+H]⁺ 897, found 897.5.

### Step 17: synthesis of trifluoroacetate salt of compound 1

Compound **1-16** (213 mg) was dissolved in tetrahydrofuran (5 mL), and triethylamine trihydrofluoride (191.35 mg) was added. The mixture was stirred at 25 °C for 16 h. After the reaction was completed, the reaction solution was concentrated to dryness under reduced pressure, and separated by high performance liquid chromatography (column: Welch Xitimate C18, length×inner diameter: 100 mm×40 mm, 3 µm; preparation method: the crude product was dissolved in dimethyl sulfoxide and filtered through a 0.45 µm filter membrane to give a sample solution; mobile phase system: acetonitrile/water (containing 0.075% trifluoroacetic acid), gradient elution method: acetonitrile, from 17% to 57% in 8 min) to give a trifluoroacetate salt of compound 1.

¹H NMR (400MHz, DMSO-*d₆*) δ: 10.86 - 10.34 (m, 2H), 9.87 (s, 1H), 9.43 - 9.01 (m, 2H), 8.15 (d, *J*=9.6 Hz, 1H), 8.07 (s, 1H), 7.47 (d, *J*=4.0 Hz, 2H), 7.31 (s, 1H), 7.15 (d, *J*=7.6 Hz, 1H), 7.07 (s, 2H), 7.03 - 6.76 (m, 3H), 6.53 (d, *J*=9.6 Hz, 1H), 6.28 (s, 1H), 5.42 (d, *J*=8.0 Hz, 1H), 4.78 (s, 3H), 4.38 (s, 2H), 3.36 (s, 4H), 3.19 (s, 2H), 3.09 (s, 2H), 2.71 (s, 3H), 2.54 (s, 1H), 2.32 (s, 2H), 2.22 (s, 2H), 1.99 (s, 4H), 1.64 (d, *J*=7.6 Hz, 2H), 1.46 (d, *J*=10.8 Hz, 2H).

MS-ESI calcd. [M+H]⁺ 783, found 783.4.

### Step 18: synthesis of the compound of fomula (I)

Compound **1-16** (6.26 g, 6.98 mmol) was dissolved in tetrahydrofuran (120 mL), and triethylamine trihydrofluoride (5.62 g, 34.89 mmol, 5.69 mL) was added. The mixture was stirred at 25 °C for 16 h. After the reaction was completed, the supernatant is discarded, and the solid was washed with 50 mL of tetrahydrofuran and concentrated to dryness under reduced pressure. The obtained solid was dispersed in a dichloromethane/methanol mixed solution (700 mL, v/v=10/1) and a saturated sodium bicarbonate solution (400 mL), and stirred for 2 h. The liquids were separated, and the organic phase was washed with 200 mL of deionized water and concentrated to dryness under reduced pressure to give the compound of formula (I).

¹H NMR (400MHz, DMSO-*d₆*) δ: 8.13 (d, *J*=10.0 Hz, 1H), 7.71 (s, 1H), 7.46 (d, *J*=4 Hz, 2H), 7.24 (br s, 1H), 7.07 - 7.04 (m, 3H), 6.98 - 6.96 (m, 2H), 6.89 (d, *J*=8.0 Hz, 1H), 6.40 (d, *J*=10.0 Hz, 1H), 5.37 (br s, 1H), 5.08 - 5.05 (m, 1H), 4.69 - 4.65 (m, 3H), 3.80 (s, 2H), 3.29 - 3.25 (m, 4H), 2.91 (br t, *J*=7.6 Hz, 2H), 2.83 - 2.66 (m, 2H), 2.42 - 2.39 (m, 4H), 2.17 - 2.12 (m, 2H), 1.99 - 1.89 (s, 7H), 1.65 (brs, 2H), 1.34 - 1.32 (m, 4H).

MS-ESI calcd. [M+H]⁺ 783, found 783.2.

### Example 1: preparation of crystal form C of the compound of formula (1)

100 mL of acetonitrile was added to 5.46 g of the compound of formula (I) prepared in step **18** of Reference Example 1, and the mixture was slurried and stirred at 25 °C for 16 h. The mixture was concentrated under reduced pressure to remove two thirds of acetonitrile, then added with 100 mL of deionized water, slurried and stirred at 25 °C for 15 min. The mixture was filtered and the solid was collected. 100 mL of acetonitrile was added to the obtained solid, and the mixture was slurried and stirred at 25 °C for 4 h. The mixture was filtered, and the solid was collected and vacuum dried at 50°C for 16 h to give the crystal form C of the compound of formula (I). Its XRPD, TGA and DSC patterns are shown in FIGs. 7-9.

### Example 2: preparation of crystal form A of the compound of formula (I)

Compound **1-16** (5 g, 5.57 mmol) was dissolved in tetrahydrofuran (100 mL), and triethylamine trihydrofluoride (4.49 g, 27.86 mmol, 4.54 mL) was added. The mixture was stirred at 25 °C for 16 h. After the reaction was completed, the supernatant is discarded, and the solid was washed with 100 mL of tetrahydrofuran and concentrated to dryness under reduced pressure. The obtained solid was dispersed in a dichloromethane/methanol mixed solution (1500 mL, v/v=10/1) and a saturated sodium bicarbonate solution (800 mL), and stirred for 2 h. The liquids were separated, and the organic phase was washed with 400 mL of deionized water and concentrated to dryness under reduced pressure to give the solid compound of formula (I). The obtained solid was stirred in 100 mL of acetonitrile at 25 °C for 16 h. The mixture was filtered, and the obtained solid was continued to stir in 100 mL of acetonitrile at 50 °C for 16 h. The mixture was filtered, and the solid was collected and vacuum dried at 50°C for 16 h to give the crystal form A of the compound of formula (I). Its XRPD, TGA and DSC patterns are shown in FIGs. 1-3.

### Example 3: preparation of crystal form E of the compound of formula (I)

0.5 mL of methanol was added to 0.05 g of the compound of formula (I) prepared in step **18** of Reference Example 1, and the mixture was slurried and stirred at 65 °C for 2 h. The mixture was filtered, and the solid was collected and vacuum dried at 40°C for 16 h to give the crystal form E of the compound of formula (I). Its XRPD, TGA and DSC patterns are shown in FIGs. 13-15.

### Example 4: preparation of crystal form F of the compound of formula (I)

2 mL of methanol was added to 0.1 g of the crystal form E of the compound of formula (I), the mixture was slurried and stirred at 65 °C for 2 h, then cooled to room temperature and filtered to give the solid. The above process was repeated three times.The mixture was filtered, and the solid was collected and vacuum dried at 40°C for 16 h to give the crystal form F of the compound of formula (I). Its XRPD, TGA and DSC patterns are shown in FIGs. 16-18.

### Example 5: Polymorph Screening of the compound of formula (I)

The crystal form C of the compound of formula (I) can be converted into various free crystal formsunder the following conditions:
Crystal form A:0.5 mL of methanol was added to about 15.2 mg of the crystal form C of the compound of formula (I), and the mixture was suspended and stirred at room temperature for about 4 days and then air-dried at room temperature to give the crystal form A.

Crystal form B: 0.5 mL of tetrahydrofuran was added to about 15.1 mg of the crystal form C of the compound of formula (I), and the mixture was suspended and stirred at 40°C for about 7 days, and then stirred with increasing and decreasing the temperature cyclically (50°C to 5°C, 0.1°C/min, 2 cycles). The mixture was dried in the open air at room temperature for 1 day to give the crystal form B. Its XRPD, TGA and DSC patterns are shown in FIGs. 4-6.

Crystal form D: 0.5 mL of tetrahydrofuran was added to about 15.0 mg of the crystal form C of the compound of formula (I), and the mixture was suspended and stirred at room temperature for about 4 days, then dried in the air at room temperature for 2 days and vacuum dried at room temperature for 5 days. (The sample became a gel after drying in the air at room temperature, and a powdery solid crystal form D was obtained after vacuum drying. Its XRPD, TGA and DSC patterns are shown in FIGs. 10-12.

### Example 6: preparation of crystal form G of the compound of formula (II)

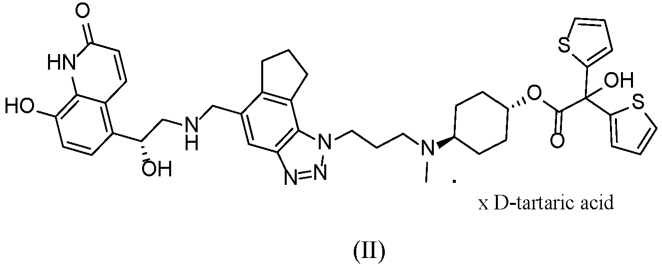

100 mg of the compound of formula (I) prepared in step **18** of Reference Example 1 was dissolved in 2 mL of a mixed solvent of dioxane/ethanol (1/1), and a solution of 19 mg of D-tartaric acid dissolved in 1 mL of ethanol was added dropwise at room temperature, and a white solid precipitated. The mixture was heated to 50-55°C and stirred for 4 h, then filtered. The solid was collected. 35 mg of the solid was taken and slurried in 3 mL of ethanol at 50-55°C for 6 h. The mixture was filtered, and the solid was collected and vacuum dried at 40°C for 14 h to give the crystal form G of the compound of formula (II). Its XRPD, TGA and DSC patterns are shown in FIGs. 19-21.

### Example 7: preparation of crystal form H of the compound of formula (III)

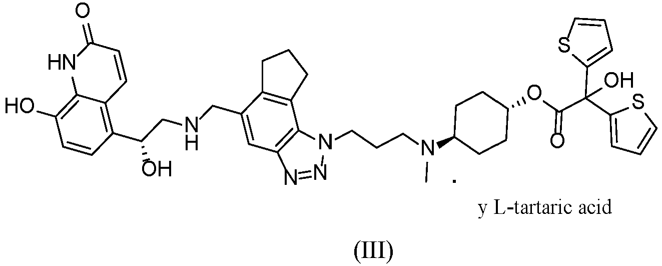

100 mg of the compound of formula (I) prepared in step **18** of Reference Example 1 was dissolved in 2 mL of a mixed solvent of methanol/tetrahydrofuran (1/1), and a solution of 19 mg of L-tartaric acid dissolved in 1 mL of methanol was added dropwise at room temperature, and a white solid precipitated. The mixture was heated to 50°C and stirred for 6 h, then filtered. The solid was collected and vacuum dried at 40°C for 14 h to give the crystal form H of the compound of formula (III). Its XRPD, TGA and DSC patterns are shown in FIGs. 22-24.

### Test Example 1: Crystal stability test of the form C of the compound of formula (I) under high humidity conditions

The crystal form C of the compound of formula (I) was placed under different humidity conditions at room temperature and then tested by TGA to preliminarily evaluate its water content changes under different humidity conditions. The results are summarized in Table 9. The results show that the TGA weight loss of the crystal form C of the compound of formula (I) increased slightly after being placed under both conditions.

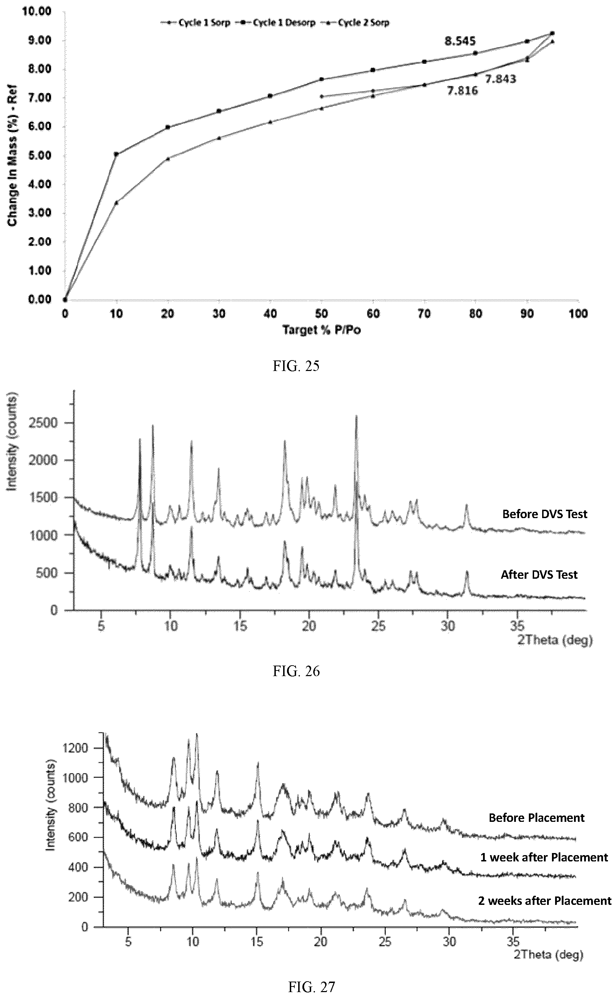

At the same time, a DVS test was performed on the crystal form C of the compound of formula (I). The result (FIG. 25 ) showed that the moisture adsorption of the sample at 25°C/80% RH was 7.84%, and the XRPD result (FIG. 26 ) showed that the crystal form of the sample remained unchanged after the DVS test.

**Table 9. TGA results of free base crystal form C after placement**

| Placement conditions | Time | TGA weight loss (%, 150 °C) |
|---|---|---|
| ~81%RH*/ room temperature | 24 hours | 10.4 |
| ~94%RH#/ room temperature | 24 hours | 9.9 |

| | | |
|---|---|---|
| *: saturated potassium bromide solution. #: saturated potassium nitrate solution. | | |

Conclusion: the crystal form C of the compound of formula (I) of the present application has good stability and druggability.

### Test Example 2: Study on crystal stability of the form E of the compound of formula (I) under high humidity conditions

The crystal form E of the compound of formula (I) was placed under 94% RH at room temperature for 1 to 2 weeks and then subjected to XRPD testing to evaluate its solid stability in the high humidity environment. The results (FIG. 27) showed that the crystal form of the sample remained unchanged after placement.

Conclusion: the crystal form E of the compound of formula (I) of the present application has good stability and druggability.

### Bioactivity Assay

### Test Example 1. Assay on Agonistic Effect of Compound on β1 Receptor by HTRF cAMP Method

### Experimental objective:

β1 receptor is a G protein-coupled receptor, which is mainly coupled with Gs protein, and can activate adenylate cyclase activity through the Gs protein after being activated by binding to the ligand, thereby increasing the level of intracellular cAMP. In this experiment, the agonistic activity of the compound against the β1 receptor *in vitro* was assayed by the cAMP kit.

### Experimental method:

β1 cells (expressing human ADRB1 gene) from ICE Bioscience Inc. were grown under standard conditions. The cells were collected and diluted with 1 × Stimulation Buffer, and 9 µL of the cell dilution was added to a white low-volume 384-well plate with 4000 cells seeded in each well. Compound was serially diluted in DMSO in a 5-fold gradient to obtain 10 concentrations. Before testing, compound that had been serially diluted in DMSO were subjected to 100-fold dilution with 1 × Stimulation Buffer to obtain compound working solutions. The concentration of the vehicle DMSO was 0.1%. Eu-cAMP and ULight^{™}-anti-cAMP were diluted to the working concentration with Detection buffer. 5 µL of Eu-cAMP diluent and 5 µL of ULight^{™}-anti-cAMP diluent were sequentially added into corresponding experiment wells. After 1 h of incubation at room temperature, readings at 665 nm and 620 nm were measured under the excitation wavelength of 330 nm using a Biotek microplate reader. The activity of the compound was obtained by plotting through Ratio (665/620) against the concentration of the compound, fitting the curve by a nonlinear regression method using GraphPad Prism 7 software and calculating EC₅₀.

The experimental results are shown in Table 10.

Table 10. Agonistic activity of the compound of the present application against the β1 receptor *in vitro*

| Compound No. | **EC**₅₀ (nM) |
|---|---|
| Trifluoroacetate salt of the compound of formula (I) | 84.54 |

Conclusion: the compound of the present application have a certain agonistic effect on the β1 receptor.

### Test Example 2. Assay on Agonistic Effect of Compound on β2 Receptor by HTRF cAMP Method

### Experimental objective:

β2 receptor is a G protein-coupled receptor, which is mainly coupled with Gs protein, and can activate adenylate cyclase activity through the Gs protein after being activated by binding to the ligand, thereby increasing the level of intracellular cAMP. In this experiment, the agonistic activity of the compound against the β2 receptor *in vitro* was assayed by the cAMP kit.

### Experimental method:

β2 cells (expressing human ADRB2 gene) from ICE Bioscience Inc. were grown under standard conditions. The cells were collected and diluted with 1 × Stimulation Buffer, and 9 µL of the cell dilution was added to a white low-volume 384-well plate with 1000 cells seeded in each well. Compound was serially diluted in DMSO in a 5-fold gradient to obtain 10 concentrations. Before testing, compound that had been serially diluted in DMSO were subjected to 100-fold dilution with 1 × Stimulation Buffer to obtain compound working solutions. The concentration of the vehicle DMSO was 0.1%. Eu-cAMP and ULight^{™}-anti-cAMP were diluted to the working concentration with Detection buffer. 5 µL of Eu-cAMP diluent and 5 µL of ULight^{™}-anti-cAMP diluent were sequentially added into corresponding experiment wells. After 1 h of incubation at room temperature, readings at 665 nm and 620 nm were measured under the excitation wavelength of 330 nm using a Biotek microplate reader. The activity of the compound was obtained by plotting through Ratio (665/620) against the concentration of the compound, fitting the curve by a nonlinear regression method using GraphPad Prism 7 software and calculating EC₅₀

The experimental results are shown in Table 11.

**Table 11. Agonistic activity of the compound of the present application against the β2 receptor in vitro**

| Compound No. | **EC**₅₀ (nM) |
|---|---|
| Trifluoroacetate salt of the compound of formula (I) | 3.472 |

Conclusion: the compound of the present application have a strong or very strong agonistic effect on the β2 receptor.

### Test Example 3. M3 Receptor Affinity Test

### Experimental objective:

M3 receptor is a G protein-coupled receptor. In this experiment, the activity of test compound against the M3 receptor *in vitro* was assayed by the competition of radioisotope labeled NMS and non-isotope labeled test compound for an M3 binding site.

### Experimental method:

The experiment was performed by a radioisotope affinity assay. The M3 receptor membrane protein was prepared at 10 µg/mL by Biology Department of WuXi AppTec, and compound was serially diluted in a 2.5-fold gradient in DMSO to obtain 10 concentrations. Radioisotope 3H-NMS was prepared at 0.2 nM in an assay buffer (10 mM HEPES, 1 mM MgCl₂, pH 7.40). In formal testing, the experimental system comprising 1 µL of the test compound, 100 µL of M3 receptor membrane protein and 100 µL of radioisotope was shaken in a shaker at 300 RPM for 2 h at room temperature. A GF/C plate (Perkin Elmer, Cat No. 6055690) was prefoamed with 0.3% PEI, and the membrane proteins in the reaction solution were collected by filtration onto the GF/C plate. The signal values were read by a Perkin Elmer Microbeta2 instrument and the inhibition rate for each concentration point was shown as a percentage. The experimental results are shown in Table 12.

**Table 12. Activity of the compound of the present application against the M3 receptor in vitro**

| Compound No. | IC₅₀ (nM) |
|---|---|
| Trifluoroacetate salt of the compound of formula (I) | 0.384 |

Conclusion: the compound of the present application have a strong or very strong binding effect on the M3 receptor.

### Test Example 4. M2 Receptor Affinity Test

### Experimental objective:

M2 receptor is a G protein-coupled receptor. In this experiment, the activity of test compound against the M2 receptor *in vitro* was assayed by the competition of radioisotope labeled NMS and non-isotope labeled test compound for an M2 binding site.

### Experimental method:

The M2 receptor membrane protein was prepared at 100 µg/mL by Biology Department of WuXi AppTec, and compound was serially diluted in a 2.5-fold gradient in DMSO to obtain 10 concentrations. Radioisotope 3H-NMS was prepared at 0.2 nM in an assay buffer (10 mM HEPES, 1 mM MgCl₂, pH 7.40). In formal testing, the experimental system comprising 1 µL of the test compound, 100 µL of M2 receptor membrane protein and 100 µL of radioisotope was shaken in a shaker at 300 RPM for 2 h at room temperature. A GF/C plate (Perkin Elmer, Cat No. 6055690) was prefoamed with 0.3% PEI, and the membrane proteins in the reaction solution were collected by filtration onto the GF/C plate. The signal values were read by a Perkin Elmer Microbeta2 instrument and the inhibition rate for each concentration point was shown as a percentage.

The experimental results are shown in Table13.

**Table 13. Activity of the compound of the present application against the M2 receptor in vitro**

| Compound No. | IC₅₀ (nM) |
|---|---|
| Trifluoroacetate salt of the compound of formula (I) | 0.06196 |

Conclusion: the compound of the present application have a strong binding effect on the M2 receptor.

## Claims

1. A crystal form of a compound of formula (I),

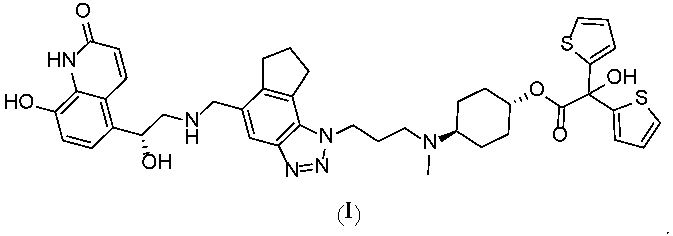

2. The crystal form of the compound of formula (I) according to claim 1, wherein the crystal form has an X-ray powder diffraction pattern comprising diffraction peaks, in terms of 2θ angle, at 12.65±0.20° and 24.06±0.20°; or, wherein the crystal form has an X-ray powder diffraction pattern as shown in FIG. 1.

3. The crystal form of the compound of formula (I) according to claim 1, wherein the crystal form has an X-ray powder diffraction pattern comprising a diffraction peak, in terms of 2θ angle, at 12.66±0.20°;
or, wherein the crystal form has an X-ray powder diffraction pattern as shown in FIG. 4.

4. The crystal form of the compound of formula (I) according to claim 1, wherein the crystal form has an X-ray powder diffraction pattern comprising diffraction peaks, in terms of 2Θ angle, at 7.77±0.20°, 8.70±0.20°, 11.49±0.20°, 18.22±0.20°, and 23.39±0.20°;
or, wherein the crystal form has an X-ray powder diffraction pattern comprising at least 5, 6, 7, or 8 diffraction peaks, in terms of 2Θ angle, selected from the group consisting of: 7.77±0.20°, 8.70±0.20°, 11.49±0.20°, 13.45±0.20°, 18.22±0.20°, 19.82±0.20°, 21.88±0.20°, and 23.39±0.20°;
or, wherein the crystal form has an X-ray powder diffraction pattern comprising diffraction peaks, in terms of 2Θ angle, at 7.77±0.20°, 8.70±0.20°, 11.49±0.20°, 13.45±0.20°, 18.22±0.20°, 19.82±0.20°, 21.88±0.20°, and 23.39±0.20°
or, wherein the crystal form has an X-ray powder diffraction pattern comprising at least 12, 13, 14, 15, or 16 diffraction peaks, in terms of 2Θ angle, selected from the group consisting of: 7.77±0.20°, 8.70±0.20°, 9.97±0.20°, 10.64±0.20°, 11.49±0.20°, 13.45±0.20°, 15.55±0.20°, 18.22±0.20°, 19.82±0.20°, 20.37±0.20°, 21.88±0.20°, 23.39±0.20°, 23.99±0.20°, 27.29±0.20°, 27.75±0.20°, and 31.35±0.20°;
or, wherein the crystal form has an X-ray powder diffraction pattern comprising diffraction peaks, in terms of 2Θ angle, at 7.77±0.20°, 8.70±0.20°, 9.97±0.20°, 10.64±0.20°, 11.49±0.20°, 13.45±0.20°, 15.55±0.20°, 18.22±0.20°, 19.82±0.20°, 20.37±0.20°, 21.88±0.20°, 23.39±0.20°, 23.99±0.20°, 27.29±0.20°, 27.75±0.20°, 31.35±0.20°;
or, wherein the crystal form has an X-ray powder diffraction pattern as shown in FIG. 7.

5. The crystal form of the compound of formula (I) according to claim 4, wherein the crystal form has a differential scanning calorimetry curve comprising an endothermic peak at 112.2°C;
or, wherein the crystal form has a differential scanning calorimetry curve pattern as shown in FIG. 9.

6. The crystal form of the compound of formula (I) according to claim 1, wherein the crystal form has an X-ray powder diffraction pattern comprising diffraction peaks, in terms of 2θ angle, at 5.56±0.20° and 13.84±0.20°; or, wherein the crystal form has an X-ray powder diffraction pattern as shown in FIG. 10.

7. The crystal form of the compound of formula (I) according to claim 1, wherein the crystal form has an X-ray powder diffraction pattern comprising diffraction peaks, in terms of 2Θ angle, at 8.53±0.20°, 9.67±0.20°, 10.31±0.20°, and 15.14±0.20°;
or, wherein the crystal form has an X-ray powder diffraction pattern comprising diffraction peaks, in terms of 2Θ angle, at 8.53±0.20°, 9.67±0.20°, 10.31±0.20°, 15.14±0.20°, and 17.03±0.20°;
or, wherein the crystal form has an X-ray powder diffraction pattern comprising at least 5, 6, 7, or 8 diffraction peaks, in terms of 2Θ angle, selected from the group consisting of: 8.53±0.20°, 9.67±0.20°, 10.31±0.20°, 11.86±0.20°, 15.14±0.20°, 17.03±0.20°, 19.07±0.20°, and 23.60±0.20°;
or, wherein the crystal form has an X-ray powder diffraction pattern comprising diffraction peaks , in terms of 2Θ angle, at 8.53±0.20°, 9.67±0.20°, 10.31±0.20°, 11.86±0.20°, 15.14±0.20°, 17.03±0.20°, 19.07±0.20°, and 23.60±0.20°;
or, wherein the crystal form has an X-ray powder diffraction pattern comprising at least 10, 11, 12, or 13 diffraction peaks, in terms of 2Θ angle, selected from the group consisting of: 8.53±0.20°, 9.67±0.20°, 10.31±0.20°, 11.86±0.20°, 15.14±0.20°, 16.67±0.20°, 17.03±0.20°, 18.50±0.20°, 19.07±0.20°, 21.12±0.20°, 23.60±0.20°, 26.61±0.20°, and 29.58±0.20°;
or, wherein the crystal form has an X-ray powder diffraction pattern comprising diffraction peaks, in terms of 2Θ angle, at 8.53±0.20°, 9.67±0.20°, 10.31±0.20°, 11.86±0.20°, 15.14±0.20°, 16.67±0.20°, 17.03±0.20°, 18.50±0.20°, 19.07±0.20°, 21.12±0.20°, 23.60±0.20°, 26.61±0.20°, and 29.58±0.20°;
or, wherein the crystal form has an X-ray powder diffraction pattern as shown in FIG. 13.

8. The crystal form of the compound of formula (I) according to claim 7, wherein the crystal form has a differential scanning calorimetry curve comprising endothermic peaks at 120.0°C, 152.7°C and 192.0°C;
or, wherein the crystal form has a differential scanning calorimetry curve pattern as shown in FIG. 15.

9. The crystal form of the compound of formula (I) according to claim 1, wherein the crystal form has an X-ray powder diffraction pattern comprising diffraction peaks , in terms of 2θ angle, at 9.50±0.20°, 15.79±0.20°, and 18.44±0.20°;
or, wherein the crystal form has an X-ray powder diffraction pattern comprising diffraction peaks, in terms of 2Θ angle, at 9.50±0.20°, 13.34±0.20°, 15.79±0.20°, 18.44±0.20°, and 24.43±0.20°;
or, wherein the crystal form has an X-ray powder diffraction pattern comprising at least 5, 6, 7, or 8 diffraction peaks, in terms of 2Θ angle, selected from the group consisting of: 9.50±0.20°, 13.34±0.20°, 15.79±0.20°, 18.44±0.20°, 19.97±0.20°, 21.27±0.20°, 21.80±0.20°, and 24.43±0.20°;
or, wherein the crystal form has an X-ray powder diffraction pattern comprising diffraction peaks, in terms of 2Θ angle, at 9.50±0.20°, 13.34±0.20°, 15.79±0.20°, 18.44±0.20°, 19.97±0.20°, 21.27±0.20°, 21.80±0.20°, and 24.43±0.20°;
or, wherein the crystal form has an X-ray powder diffraction pattern as shown in FIG. 16.

10. A tartrate of the compound of formula (I) or a crystal form thereof.

11. The tartrate of the compound of formula (I) or the crystal form thereof according to claim 10, wherein the tartrate of the compound of formula (I) or the crystal form thereof is the compound of formula (II) or the crystal form thereof, 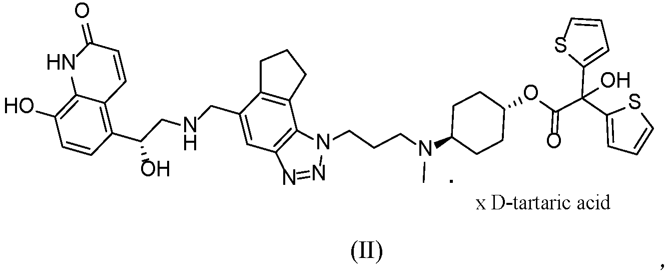
wherein, x is 0.9 to 1.1, preferably 0.9, 1 or 1.1;
or, wherein the tartrate of the compound of formula (I) or the crystal form thereof is the compound of formula (III) or the crystal form thereof, 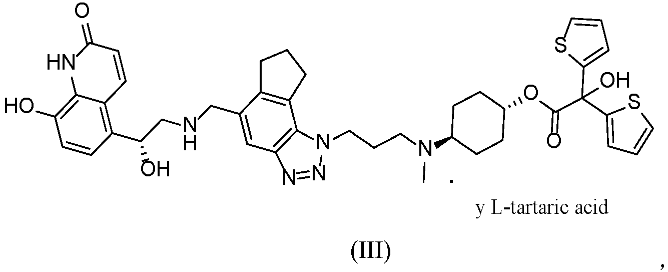
wherein, y is 0.9 to 1.1, preferably 0.9, 1 or 1.1.

12. The crystal form of the compound of formula (II) according to claim 11, wherein the crystal form has an X-ray powder diffraction pattern comprising diffraction peaks, in terms of 2Θ angle, at 11.64±0.20°, 18.51±0.20°, and 22.68±0.20°;
or, wherein the crystal form has an X-ray powder diffraction pattern comprising diffraction peaks , in terms of 2Θ angle, at 4.62±0.20°, 11.64±0.20°, 13.24±0.20°, 18.51±0.20°, and 22.68±0.20°;
or, wherein the crystal form has an X-ray powder diffraction pattern comprising at least 6, 7, or 8 diffraction peaks, in terms of 2θ angle, selected from the group consisting of: 4.62±0.20°, 10.98±0.20°, 11.64±0.20°, 13.24±0.20°, 16.76±0.20°, 18.51±0.20°, 22.68±0.20°, and 23.52±0.20°;
or, wherein the crystal form has an X-ray powder diffraction pattern comprising diffraction peaks, in terms of 2Θ angle, at 4.62±0.20°, 10.98±0.20°, 11.64±0.20°, 13.24±0.20°, 16.76±0.20°, 18.51±0.20°, 22.68±0.20°, and 23.52±0.20°;
or, wherein the crystal form has an X-ray powder diffraction pattern comprising at least 12, 13, 14, 15, or 16 diffraction peaks, in terms of 2Θ angle, selected from the group consisting of: 4.62±0.20°, 9.80±0.20°, 10.98±0.20°, 11.64±0.20°, 12.63±0.20°, 13.24±0.20°, 13.79±0.20°, 15.28±0.20°, 16.76±0.20°, 18.51±0.20°, 19.62±0.20°, 21.62±0.20°, 22.68±0.20°, 23.52±0.20°, 25.04±0.20°, and 26.64±0.20°;
or, wherein the crystal form has an X-ray powder diffraction pattern comprising diffraction peaks, in terms of 2Θ angle, at 4.62±0.20°, 10.98±0.20°, 11.64±0.20°, 13.24±0.20°, 13.79±0.20°, 15.28±0.20°, 16.76±0.20°, 18.51±0.20°, 19.62±0.20°, 22.68±0.20°, 23.52±0.20°, and 26.64±0.20°;
or, wherein the crystal form has an X-ray powder diffraction pattern comprising diffraction peaks , in terms of 2Θ angle, at 4.62±0.20°, 9.80±0.20°, 10.98±0.20°, 11.64±0.20°, 12.63±0.20°, 13.24±0.20°, 13.79±0.20°, 15.28±0.20°, 16.76±0.20°, 18.51±0.20°, 19.62±0.20°, 21.62±0.20°, 22.68±0.20°, 23.52±0.20°, 25.04±0.20°, and 26.64±0.20°;
or, wherein the crystal form has an X-ray powder diffraction pattern as shown in FIG. 19.

13. The crystal form of the compound of formula (III) according to claim 11, wherein the crystal form has an X-ray powder diffraction pattern comprising diffraction peaks, in terms of 2θ angle, at 11.56±0.20°, 18.64±0.20°, and 22.52±0.20°;
or, wherein the crystal form has an X-ray powder diffraction pattern comprising diffraction peaks, in terms of 2Θ angle, at 10.01±0.20°, 11.56±0.20°, 13.07±0.20°, 18.64±0.20°, and 22.52±0.20°;
or, wherein the crystal form has an X-ray powder diffraction pattern comprising at least 5, 6, 7, or 8 diffraction peaks, in terms of 2θ angle, selected from the group consisting of: 10.01±0.20°, 11.56±0.20°, 13.07±0.20°, 14.17±0.20°, 18.64±0.20°, 20.06±0.20°, 22.52±0.20°, and 23.48±0.20°;
or, wherein the crystal form has an X-ray powder diffraction pattern comprising diffraction peaks, in terms of 2Θ angle, at 10.01±0.20°, 11.56±0.20°, 13.07±0.20°, 14.17±0.20°, 18.64±0.20°, 20.06±0.20°, 22.52±0.20°, and 23.48±0.20°;
or, wherein the crystal form has an X-ray powder diffraction pattern comprising at least 12, 13, 14, 15, or 16 diffraction peaks, in terms of 2Θ angle, selected from the group consisting of: 4.29±0.20°, 10.01±0.20°, 10.89±0.20°, 11.56±0.20°, 13.07±0.20°, 14.17±0.20°, 14.80±0.20°, 15.66±0.20°, 16.37±0.20°, 18.64±0.20°, 20.06±0.20°, 22.52±0.20°, 23.48±0.20°, 24.80±0.20°, 25.98±0.20°, and 29.35±0.20°;
or, wherein the crystal form has an X-ray powder diffraction pattern comprising diffraction peaks, in terms of 2Θ angle, at 4.29±0.20°, 10.01±0.20°, 10.89±0.20°, 11.56±0.20°, 13.07±0.20°, 14.17±0.20°, 16.37±0.20°, 18.64±0.20°, 20.06±0.20°, 22.52±0.20°, 23.48±0.20°, and 25.98±0.20°;
or, wherein the crystal form has an X-ray powder diffraction pattern comprising diffraction peaks, in terms of 2Θ angle, at 4.29±0.20°, 10.01±0.20°, 10.89±0.20°, 11.56±0.20°, 13.07±0.20°, 14.17±0.20°, 14.80±0.20°, 15.66±0.20°, 16.37±0.20°, 18.64±0.20°, 20.06±0.20°, 22.52±0.20°, 23.48±0.20°, 24.80±0.20°, 25.98±0.20°, 29.35±0.20°;
or, wherein the crystal form has an X-ray powder diffraction pattern as shown in FIG. 22.

14. A crystal composition comprising the crystal form of the compound of formula (I) according to any one of claims 1-9, or the tartrate of the compound of formula (I) or the crystal form thereof according to any one of claims 10-13.

15. A pharmaceutical composition comprising the crystal form of the compound of formula (I) according to any one of claims 1-9, the tartrate of the compound of formula (I) or the crystal form thereof according to any one of claims 10-13, or the crystal composition according to claim 14.

16. Use of the crystal form of the compound of formula (I) according to any one of claims 1-9, the tartrate of the compound of formula (I) or the crystal form thereof according to any one of claims 10-13, the crystal composition according to claim 14, or the pharmaceutical composition according to claim 15 in preparing a medicament for treating a chronic obstructive pulmonary disease.

17. A method for preparing the crystal form of the compound of formula (I) according to claim 4, comprising a step of slurrying the compound of formula (I) in a mixed solvent of acetonitrile and water;
or, further comprising a step of separating;
or, the method comprises the following steps:
(1) adding acetonitrile to the compound of formula (I), slurrying and stirring;
(2) adding water, slurrying and stirring;
(3) filtering and collecting the solid;
(4) washing the solid obtained in step (3) with acetonitrile; and
(5) drying.

18. A method for preparing the crystal form of the compound of formula (I) according to claim 7, comprising a step of slurrying the compound of formula (I) in methanol;
or, further comprising a step of separating;
or, the method comprises the following steps:
(1) slurrying and stirring the compound of formula (I) in methanol; and
(2) filtering and drying.
